# EUROPEAN PATENT SPECIFICATION

(11) **EP 2 229 411 B1**
(45) Date of publication and mention of the grant of the patent: **27.02.2019**
(21) Application number: 08859772.9
(22) Date of filing: 12.12.2008
(51) Int. Cl.: C07K 17/02, A61K 48/00, A61K 49/00, A61K 51/04, A61P 35/00, C12N 15/10, C12N 15/11, G01N 33/566, G01N 33/574, C07K 14/485, C07K 14/71, C12N 15/87, A61K 47/62

(54) **HIGH-DENSITY LIPOPROTEIN-LIKE PEPTIDE-PHOSPHOLIPID SCAFFOLD ("HPPS") NANOPARTICLES**
HIGH-DENSITY LIPOPROTEIN-LIKE PEPTIDE-PHOSPHOLIPID SCAFFOLD-(HPPS-)NANOPARTIKEL
NANOPARTICULES DE HAUTE DENSITE À BASE DE PHOSPHOLIPIDES-PEPTIDES DE TYPE ALPHA-LIPOPROTEINES (HPPS)

(30) Priority: 12.12.2007 US 13233 P; 10.10.2008 US 104506 P
(43) Date of publication of application: 22.09.2010
(73) Proprietor: University Health Network, Toronto, ON M5G 2C4 (CA)
(72) Inventor: ZHENG, Gang, Toronto, Ontario M2N 4M8 (CA); ZHANG, Zhihong, Toronto, Ontario M4R 1V2 (CA); CORBIN, Ian, Toronto, Ontario M3H 6C1 (CA); CHEN, Juan, Toronto, Ontario M4R 1V2 (CA)
(74) Representative: Elsy, David
(86) International application number: PCT/CA2008/002203
(87) International publication number: WO 2009/073984

(56) References cited:
- WO-A1-02/15923
- WO-A1-2005/070400
- WO-A1-2007/051303
- WO-A1-2007/145659
- WO-A1-2007/145659
- WO-A2-2004/050062
- WO-A2-2006/073419
- JUAN CHEN ET AL: "Ligand Conjugated Low-Density Lipoprotein Nanoparticles for Enhanced Optical Cancer Imaging in Vivo", JOURNAL OF THE AMERICAN CHEMICAL SOCIETY, vol. 129, no. 18, 1 May 2007 (2007-05-01), pages 5798-5799, XP055162857, ISSN: 0002-7863, DOI: 10.1021/ja069336k
- IAN R. CORBIN ET AL: "Low-Density Lipoprotein Nanoparticles as Magnetic Resonance Imaging Contrast Agents", NEOPLASIA, vol. 8, no. 6, 1 June 2006 (2006-06-01), pages 488-498, XP055162863, ISSN: 1476-5586, DOI: 10.1593/neo.05835
- GANG ZHENG ET AL: "Rerouting Lipoprotein Nanopartilces to Selected Altemate Receptors for the Targeted Delivery of Cancer Diagnostic and Therapeutic Agents", PROCEEDINGS OF THE NATIONAL ACADEMY OF SCIENCES, NATIONAL ACADEMY OF SCIENCES, US, vol. 102, no. 49, 1 January 2005 (2005-01-01), pages 17757-17762, XP008137631, ISSN: 0027-8424, DOI: 10.1073/PNAS.0508677102
- JUAN C. FRIAS ET AL: "Recombinant HDL-Like Nanoparticles: A Specific Contrast Agent for MRI of Atherosclerotic Plaques", JOURNAL OF THE AMERICAN CHEMICAL SOCIETY, vol. 126, no. 50, 1 December 2004 (2004-12-01), pages 16316-16317, XP055163160, ISSN: 0002-7863, DOI: 10.1021/ja044911a
- MOHAMAD NAVAB ET AL: "Apolipoprotein A-I Mimetic Peptides", ARTERIOSCLEROSIS, THROMBOSIS, AND VASCULAR BIOLOGY, LIPPINCOTT WILLIAMS & WILKINS, US, vol. 25, no. 7, 14 April 2005 (2005-04-14) , pages 1325-1331, XP008137635, ISSN: 1079-5642, DOI: 10.1161/01.ATV.0000165694.39518.95
- CORBIN, IR ET AL.: 'Enhanced Cancer-Targeted Delivery Using Engineered High-Density Lipoprotein-Based Nanocarriers.' J. BIOMED. NANOTECHOL. vol. 3, no. 4, December 2007, pages 367 - 376, XP008137630
- RENSEN, PCN ET AL.: 'Recombinant Lipoproteins: Lipoprotein-Like Lipid Particles for Drug Targeting.' ADV. DRUG DELIV. REV. vol. 47, 2001, pages 251 - 276, XP002273271
- DE VRUEH, RLA ET AL.: 'Carrier-Mediated Delivery of 9-(2- phosphonylmethoxyethyl) Adenine to Parenchymal Liver Cells: a Novel Therapeutic Approach for Hepatitis B.' ANTIMICROB. AGENTS CHEMOTHER. vol. 44, no. 3, 2000, pages 477 - 483, XP008137634
- ZHENG, G ET AL.: 'Rerouting Lipoprotein Nanopartilces to Selected Altemate Receptors for the Targeted Delivery of Cancer Diagnostic and Therapeutic Agents.' PROC. NATL. ACAD. SCI. USA. vol. 102, no. 49, 2005, pages 17757 - 17762, XP008137631
- NAVAB, M ET AL.: 'Apolipoprotein A-I Mimetic Peptides.' ARTERIOSCLER. THROMB. VASC. BIOL. vol. 25, no. 7, 2005, pages 1325 - 1331, XP008137635

## Description

### Background of the Invention

### Field of the Invention

The invention relates to a peptide-stabilizing ultra-small nanoparticle-based drug delivery system that allows targeted delivery of active agents for the detection and treatment of cancers and other diseases. The active agents can be located in the core or the surface of the nanoplatform, whereas cell surface receptor ligands are attached to the surface of the nanoplatform.

### Background Art

### Nanoplatform

Nanoplatforms are nanoscale structures that are designed as general platforms to create a diverse set of multifunctional diagnostic and therapeutic devices. Such nanoscale devices typically have dimensions smaller than 100 nm and thus are comparable in size to other biological entities. They are smaller than human cells (human cells are 10,000 to 20,000 nm in diameter) and organelles and similar in size to large biological macromolecules such as enzymes and receptors. Hemoglobin, for example, is approximately 5 nm in diameter, while the lipid bilayer surrounding cells is on the order of 6 nm thick. Nanoscale devices smaller than 50 nm can easily enter most cells, while those smaller than 20 nm can transit out of blood vessels (NIH/NCI Cancer Nanotechnology. NIH Publication No 04-5489 (2004)). As a result, nanodevices can readily interact with biomolecules both on the cell surface and within the cell, often in ways that do not alter the behavior and biochemical properties of those molecules. Thus, nanodevices offer an entirely unique vantage point from which to view and manipulate fundamental biological pathways and processes. Most of the multifunctional nanoplatforms reported so far are made of synthetic nanostructures such as dendrimers (spherical, branched polymers) (Quintana, A. et al. Journal of the American Chemical Society. 2003 125(26):7860-5*),* polymeric (Xu, H., Aylott, J.W. & Kopelman, R. Analyst. 2002 Nov;127(11):1471-7*,* Pan, D., Turner, J.L. & Wooley, K.L. Chemical Communications, 2400-2401 (2003)) and ceramic (Kasili, P.M., Journal of the American Chemical Society. 2004 126(9):2799-806*,* Ruoslahti, E. Cancer Cell. 2002; 2(2):97-8) nanoparticles, perfluorocarbon emulsions (Anderson, S.A. et al., Magnetic Resonance in Medicine. 2000 Sep;44(3):433-9) and cross-linked liposomes (Hood, J.D. et al., Science. 2002; 296(5577):2404-7, Li, L. et al., International Journal of Radiation Oncology, Biology, Physics. 2004 15;58(4):1215-27).

One common concern among these synthetic nanoplatform designs is a biocompatibility issue, which is closely associated with short-term and long-term toxicity. Natural nanostructures could offer a solution to this biocompatibility problem. However, nanoplatforms made of naturally occurring nanostructures are rare.

Some features for an ideal nanoplatform are as follows: 1) uniform size distribution (<100 nm); 2) large payload; 3) multivalency for high binding affinity; 4) platform technologies via modularity and multifunctionality; 5) stable and long circulating time; and 6) biocompatible, biodegradable and nontoxic. Although some of the existing synthetic nanoplatforms meet some of these criteria, one common concern among these nanoplatform designs is the biocompatibility issue, which is closely associated with short-term and long-term toxicity. Natural nanostructures could offer a solution to the biocompatibility problem. However, nanoplatforms made of naturally occurring nanostructures are rare. In one example, empty RNA virus capsules from cowpea mosaic virus and flockhouse virus served as potential nanodevices (Raja K.S. et al., Biomacromolecules 2003; 4(3):472-6). The premise is that 60 copies of coat protein that assemble into a functional virus capsule offer a wide range of chemical functionality that could be used to attach homing molecules - such as monoclonal antibodies or cancer cell-specific receptor antagonist and reporter molecules - such as magnetic resonance imaging (MRI) contrast agents to the capsule surface, and to load therapeutic agents inside the capsule. Unfortunately, even though the human body has quite a tolerance for viruses of plant origin; the immunologic effect associated with these nanodevices is undesirable.

In another example, lipoprotein nanoplatforms are used for targeted delivery of active agents (PCT Application Publication No. WO 2006/073419 (publication date: July 13, 2006)). Corbin et al., Neoplasia 2006; 8(6): 488-98, Navalo et al., Arteriosclerosis, Thrombosis and Vascular Biology 2005; 25(7): 1325-31 and Corbin et al., J. Biomed. Nanotech 2007; 3(4): 367-76 all disclose nanoplatforms but none disclose one capable of being taken up by cells through the SR-B1 receptor-medicated pathway and comprising the components of the present invention.

### Targeted Delivery

Although the effect of a particular pathology often is manifest throughout the body of the afflicted person, generally, the underlying pathology may affect only a single organ or tissue. It is rare, however, that a drug or other treatment will target only the diseased organ or tissue. More commonly, treatment results in undesirable side effects due, for example, to generalized toxic effects throughout the patient's body. It would be desirable to selectively target organs or tissues, for example, for treatment of diseases associated with the target organ or tissue. It is also desirable to selectively target cancerous tissue in the body versus normal tissue.

Most therapeutic substances are delivered to the target organ or tissue through the circulation. The endothelium, which lines the internal surfaces of blood vessels, is the first cell type encountered by a circulating therapeutic substance in the target organ or tissue. These cells provide a target for selectively directing therapies to an organ or tissue.

Endothelium can have distinct morphologies and biochemical markers in different tissues. The blood vessels of the lymphatic system, for example, express various adhesion proteins that serve to guide lymphocyte homing. For example, endothelial cells present in lymph nodes express a cell surface marker that is a ligand for L-selectin and endothelial cells in Peyer's patch venules express a ligand for the α₄β₇ integrin. These ligands are involved in specific lymphocyte homing to their respective lymphoid organs. Thus, linking a drug to L-selectin or to the α₄β₇ integrin may provide a means for targeting the drug to diseased lymph nodes or Peyer's patches, respectively, provided that these molecules do not bind to similar ligands present in a significant number of other organs or tissues. Certain observations of lymphocyte circulation suggest that organ and tissue specific endothelial markers exist. Similarly, the homing or metastasis of particular types of tumor cells to specific organs or tissues further suggests that organ and tissue specific markers exist.

Targeted delivery to specific tissues, including cancerous tissue, is needed to eliminate undesirable side effects associated with unspecific delivery.

Recognizing the urgent need for delivery vehicles that specifically target desired tissues, the inventors of the present application have developed a unique peptide- stabilizing ultra-small nanoparticle-based drug delivery system whose versatility makes it useful for a variety of applications.

### Summary of the Invention

In accordance with one aspect, there is provided a non-naturally occurring peptide-lipid nanoscaffold capable of being taken up by cells through the SR-B1 receptor-mediated pathway comprising at least one phospholipid, at least one unsaturated lipid ester and at least one peptide between 6 and 30 amino acids in length, the peptide comprising an amino acid sequence capable of forming at least one amphipathic α-helix; wherein the foregoing components associate to form the peptide-phospholipid nanoscaffold. In particular embodiments, the peptide is selected from the group consisting of Class A, H, L and M amphipathic α-helices, fragments thereof, and peptides comprising a reversed peptide sequence of the Class A, H, L and M amphipathic α-helices, or fragments thereof.

Preferably, the peptide-lipid nanoscaffold further comprises at least one homing molecule, hi one aspect, the homing molecule may be covalently bound to one of the at least one peptide (preferably at an amino acid, particularly Lysine, at or near the transition between a hydrophilic face and a hydrophobic face of the amphipathic α-helix) and the at least one lipid. In another aspect, the peptide-lipid nanoscaffold comprises at least one active agent.

In one aspect, the at least one homing molecule is at least one active cell surface receptor ligand.

Also disclosed, is a pharmaceutical formulation comprising the non-naturally occurring peptide-lipid described herein.

Further disclosed, is a method of making the non-naturally occurring peptide-lipid nanoscaffold described herein, comprising combining the components thereof under conditions suitable to form the peptide-lipid nanoscaffold.

### Brief Description of the Drawings

Figure 1 Schematic of an embodiment of a peptide-stabilizing ultra-small nanoparticle-based drug delivery system of the present invention.
Figure 2 Compounds used for incorporation into the HPPS nanoparticle for diagnostic and/or therapeutic applications.
Figure 3 Synthetic protocol for preparation of *EGFp-(DiR-BOA)-HPPS (Type 1 EGFR-targeted HPPS).*
Figure 4 Synthetic protocol for preparation of *(DiR-BOA) HPPS (Type 2 EGFR-targeted HPPS).*
Figure 5 FPLC, DLS and CD of untargeted DiR-BOA loaded HPPS.
Figure 6 FPLC profile and TEM of EGFp(DIR-BOA)-HPPS .
Figure 7 Influence of DiR-BOA loading on HPPS size. A: FPLC profiles of preparation #1 and #2; B: fractions collected from FPLC. C: Correlation between HPPS size and cargo payload (R=0.91).
Figure 8 (a) FPLC profile and (b) TEM of (DiR-BOA)HPPS formed with 4F and -4F.
Figure 9 Confocal imaging studies of EGFp(DiR-BOA)-HPPS.
Figure 10 Time-dependent uptake of EGFp(DiR-BOA)-HPPS by flow cytometry.
Figure 11 Inhibition studies of EGFp(DiR-BOA)HPPS.
Figure 12 In vivo imaging studies of EGFp(DiR-BOA)-HPPS.
Figure 13 Time-dependent uptake of EGFp-Lipid (DiR-BOA) HPPS in A549 vs H520 cells by flow cytometry.
Figure 14 Flow cytometry studies of time-dependent uptake of EGFp-lipid(DiR-BOA)HPPS in cell lines with different levels of EGFR expression.
Figure 15 Flow cytometry studies of uptake inhibition of EGFp(DiR-BOA)HPPS and EGFp-lipid(DiR-BOA)HPPS by EGFp.
Figure 16 Preliminary toxicity studies of EGFp-lipid (DiR-BOA) HPPS studied by MTT and flow cytometry. a) Cell viability studied by MTT assay showing no toxicity at even 6-fold of HPPS control (standard dose used in all previous in vitro studies); b) Cell viability studied by flow cytometry showing no toxicity at 6-fold of HPPS control; c) HPPS cellular uptake studied by flow cytometry showing uptake saturation at 2-fold of HPPS control (standard dose).
Figure 17 In vivo imaging of MT1 tumor xenograft with EGFp-Lipid (DiR-BOA) HPPS (i.v.). Arrow indicates tumor.
Figure 18 *In vivo* imaging of EGFp-Lipid (DiR-BOA) HPPS in mice with human lung primary tumor xenografts, demonstrating the EGFR-targeting specificity.
Figure 19 Confocal imaging studies with folate receptor-targeted HPPS in KB (folate receptor positive) and HT1080 (folate receptor negative) cancer cells. Note: Green is from DiR-BOA fluorescence and blue is from DAPI nucleus staining).
Figure 20 Multi-component fluorescent labeling. Each component of the HPPS particle (peptide, phophospholipid, core cargo) can be tagged with a fluorescent label for detailed particle characterization and evaluation of particle degradation.
Figure 21
   (a) Western blot analysis of SR-B1 receptor content of ldlA(mSR-B1) and LdLA7 cells.
   (b) Specificity of (DiR-BOA)HPPS for SR-B1 receptor. Confocal images show strong uptake of (DiR-BOA)HPPS in SR-B1 positive cells (ldlA(mSR-B1)) but not in SR-B1 negative cells (LDLA7). Furthermore, excess HDL effectively inhibits (DiR-BOA) HPPS uptake in ldlA(mSR-B1) cells but has no effect on uptake in LDLA7.

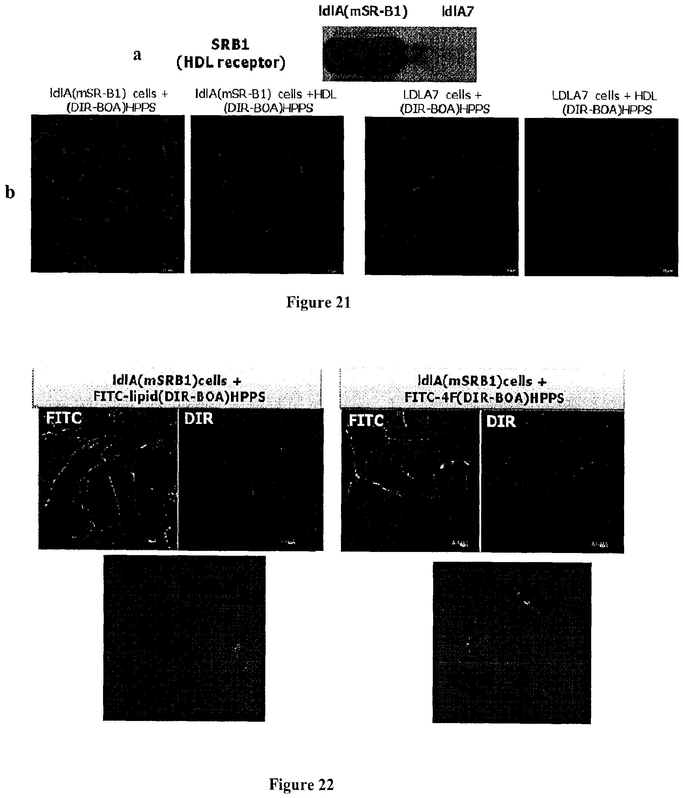

Figure 22 Confocal imaging studies with FITC-lipid(DiR-BOA)HPPS and FITC-(-4F)(DiR-BOA)HPPS and ldlA(mSR-B1) cells.

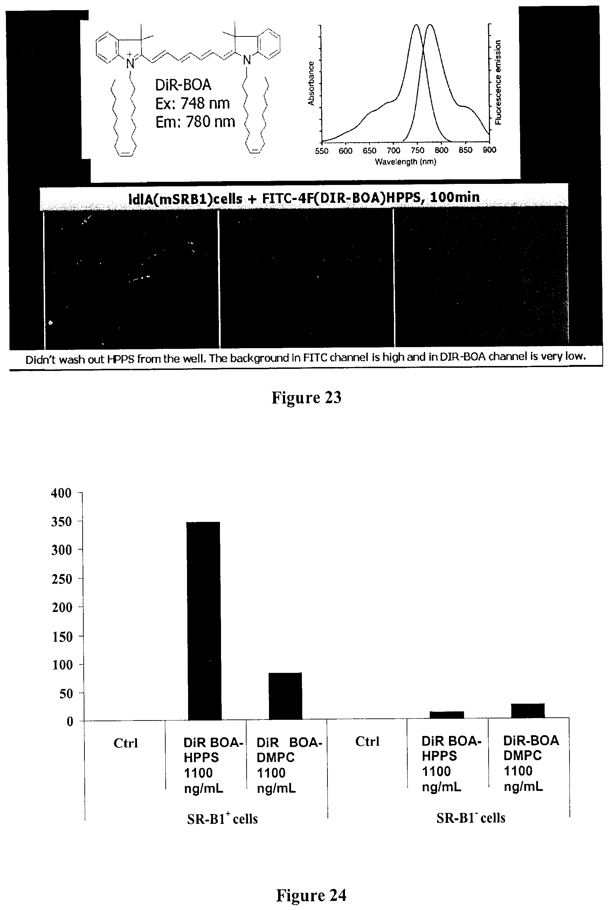

Figure 23 Confocal imaging studies with FITC-(-4F)(DiR-BOA)HPPS and ldlA(mSR-B1) cells.
Figure 24 Flow cytometry studies were used to evaluate (DiR-BOA)HPPS uptake in SR-B1⁺ and SR-B1⁻ cell lines.

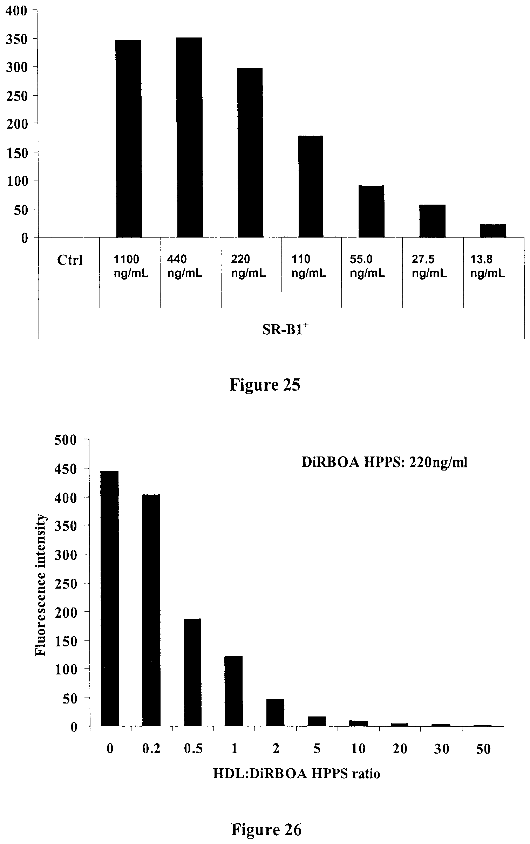

Figure 25 Flow cytometry studies were used to evaluate (DiR-BOA)HPPS concentration-dependent uptake in an SR-B1⁺ cell line.
Figure 26 Flow cytometry studies were used to evaluate HDL concentration-dependent inhibition of (DiR-BOA)HPPS uptake in an SR-B1⁺ cell line.

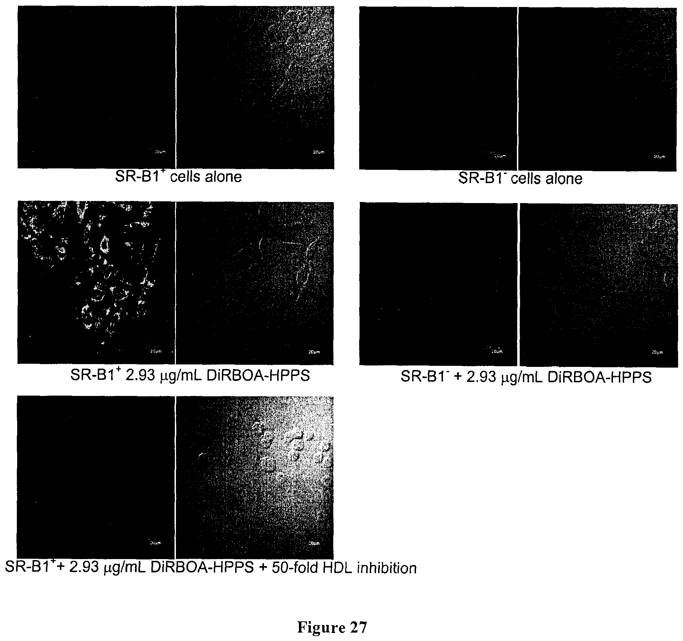

Figure 27 Flow cytometry studies were used to evaluate differential uptake of (DiR-BOA)HPPS in SR-B1⁺ and SR-B1⁻ cell lines and inhibition of uptake of (DiR-BOA)HPPS by HDL in the SR-B1⁺ cell line.

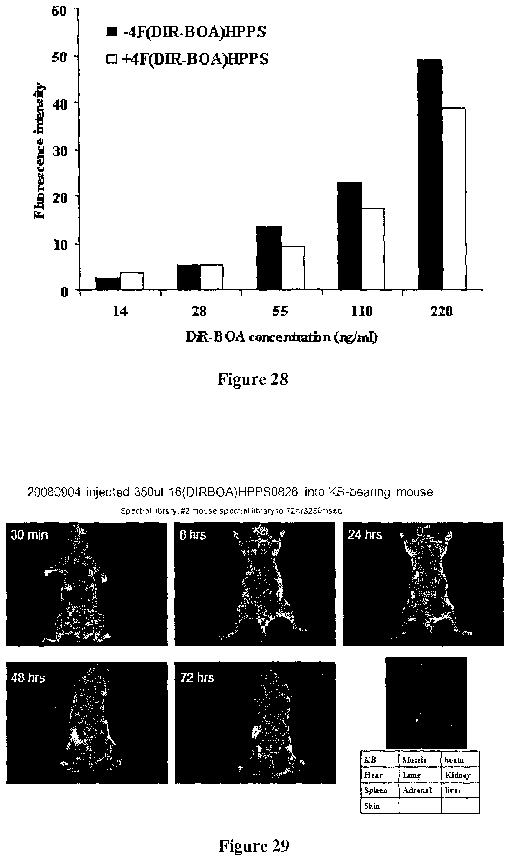

Figure 28 Flow cytometry was used to evaluate the uptake of (-4F)(DiR-BOA)HPPS versus (4F)(DiR-BOA)HPPS by ldlA(mSR-B1) cells.
Figure 29 *In vivo* imaging was performed on nude mice bearing human KB (SR-BI⁺) tumor xenograft to study the localization of -4F(DiR-BOA)HPPS.
Figure 30
   (a) Confocal studies were performed to evaluate differential uptake of (-4F)EGF(DiR-BOA)HPPS and (-4F) (DiR-BOA)HPPS in A549 and H520 cells.
   (b) Western blot analysis of surface receptor profiles of A549 and H520 cells.

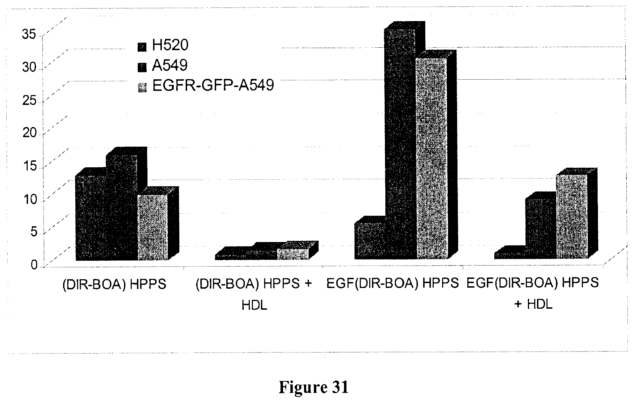

Figure 31 Flow cytometry was used to evaluate the uptake of (DiR-BOA)HPPS and EGF(DiR-BOA)HPPS (1.0 µM) by H520, A549 and EGFR-GFP-A549 cells.

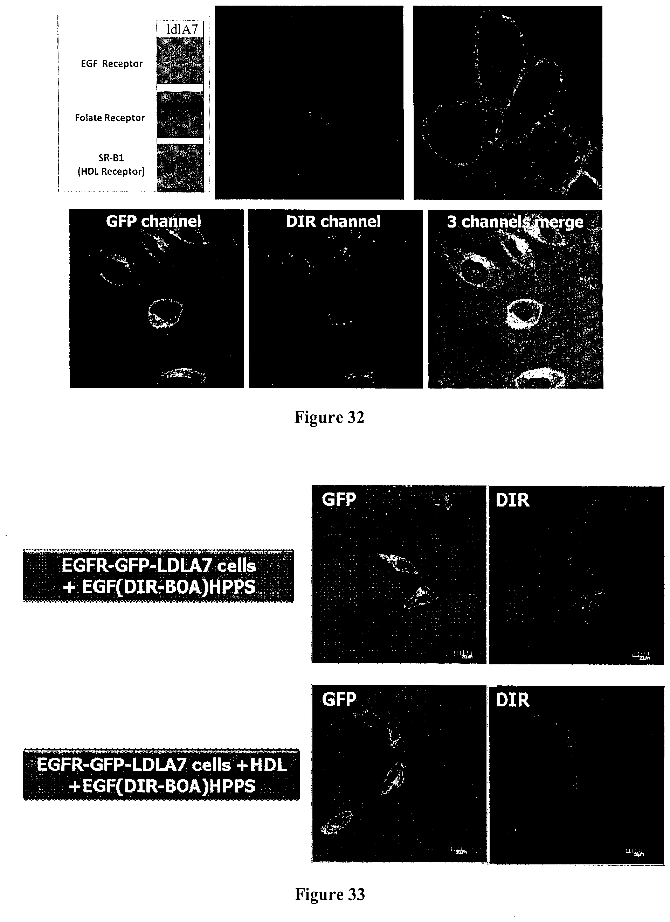

Figure 32 EGF-HPPS studies were performed in LDLA7 cells transfected with the fusion gene of EGFR-green fluorescent protein (GFP). This experiment removes any contribution SR-B1 may make to the cellular uptake of EGF-HPPS. Following a 3 hour incubation with (-4F) EGF(DiR-BOA)HPPS, EGFR-GFP-LDLA7 cells displayed a strong signal of DiR-BOA within the cytosol, indicating avid uptake of EGF-HPPS through EGFR. The GFP signal for the EGFR was visualized on the outer plasma membrane.
Figure 33 Confocal studies were performed to evaluate the effect of HDL on the uptake of (-4F)EGF(DiR-BOA)HPPS in EGFR-GFP-LDLA7 cells.

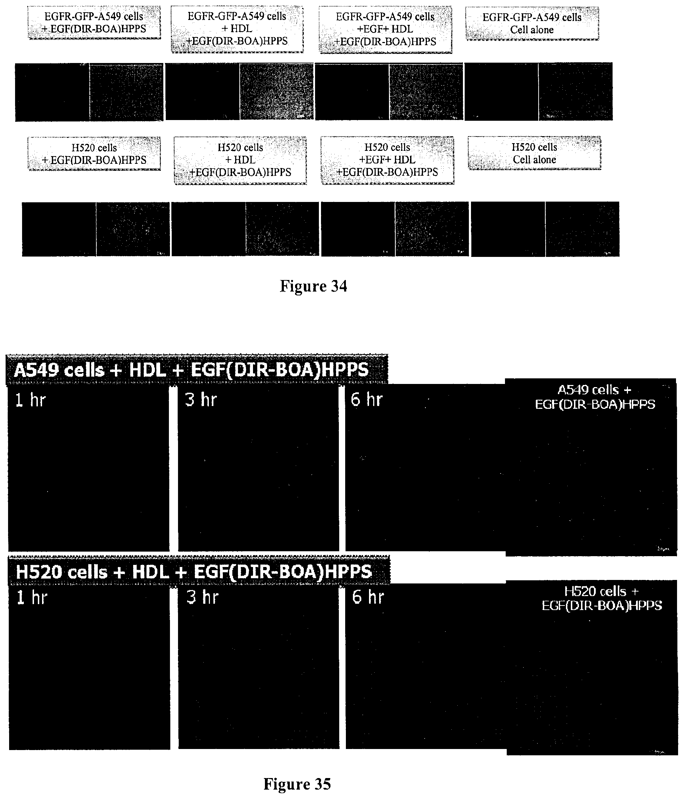

Figure 34 Confocal imaging of EGFR-GFP-A549 and H520 cells incubated with EGF(DiR-BOA)HPPS.
Figure 35 Confocal studies were performed to evaluate the effect of HDL on the uptake of (-4F)EGF(DiR-BOA)HPPS in A549 and H520 cells.

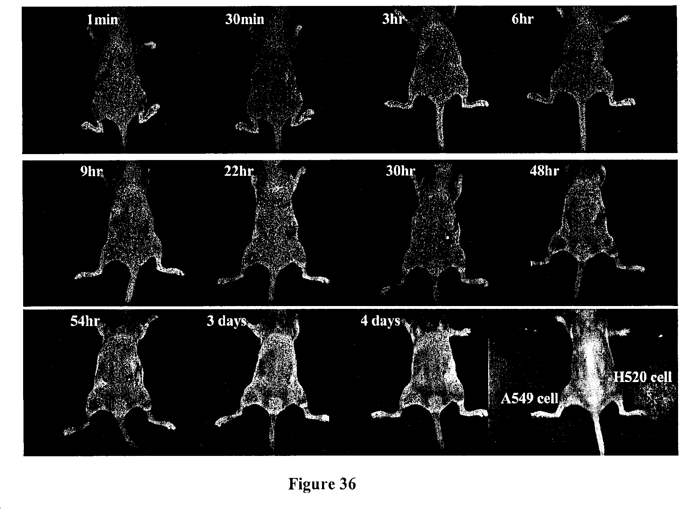

Figure 36 *In vivo* imaging was performed on nude mice bearing dual tumor (A549 and H520) to determine the targeting ability of (-4F)EGF(DiR-BOA)HPPS.

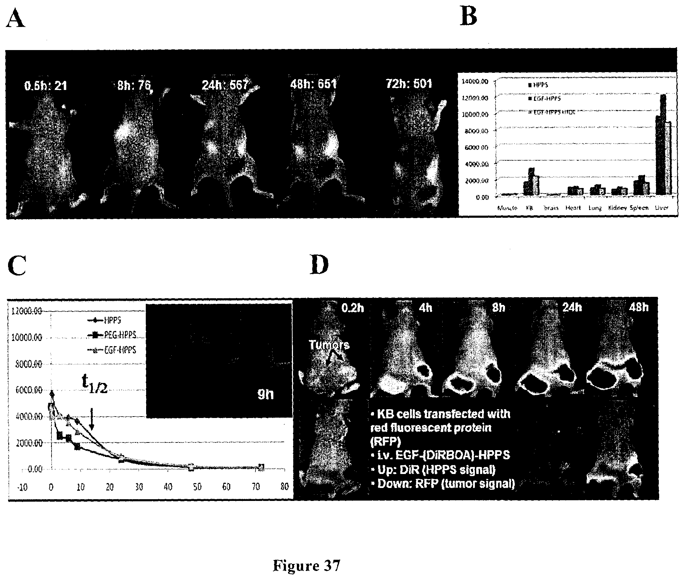

Figure 37. *In vivo* validation of (-4F)EGF(DiR-BOA)HPPS. In vivo imaging (A) and biodistribution (B) shows improved delivery specificity and efficacy of HPPS. C) HPPS matches HDL's long circulation time (t1/2∼14h). D) Accumulation of HPPS fluorescence signal (DiRBOA) in tumor tissues matches tumor cells fluorescence (transfected with red fluorescence protein).

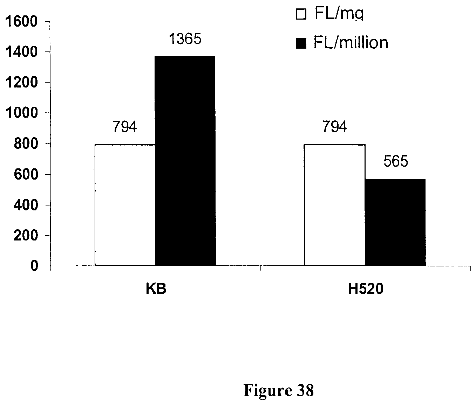

Figure 38 Cellular uptake of (-4F)EGF(DiR-BOA)HPPS in KB tumor and H520 tumor. White bars represent fluorescence units per mg of tissue and the black bars represent fluorescence units per million cells. The data reflects the average from two tumor-bearing mice.

### Detailed Description of the Present Invention

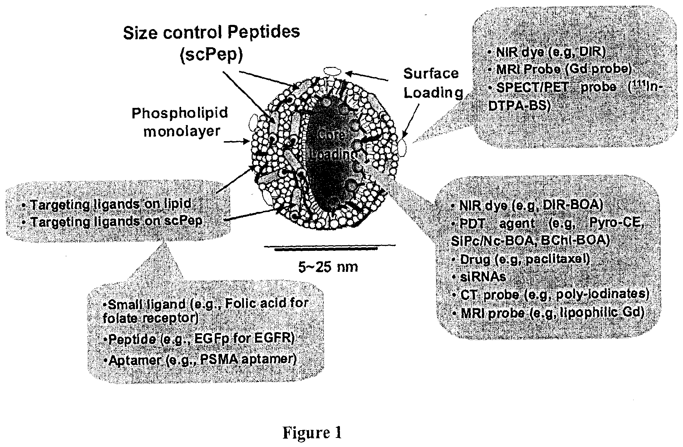

Figure 1 demonstrates an embodiment of the present invention. Specifically, Figure 1 illustrates a peptide-stabilizing ultra-small nanoparticle-based drug delivery system, called "HPPS" (High-density lipoprotein-like Peptide-Phospholipid Scaffold), that allows targeted delivery of active agents for the detection and treatment of cancers and other diseases. The HPPS nanoparticles comprise three components: 1) specific amphipathic size-control peptides (scPep) and phospholipids which form an ultra-small nanocarrier (5 to 25nm), 2) disease-specific targeting ligands which may be conjugated directly to the scPep on the nanocarrier, conjugated to surface phospholipid of the nanocarrier, or disease-specific targeting ligands such as proteins or fragments thereof which may be incorporated into the nanocarriers without conjugation to scPep or phospholipids (not shown in Figure 1), and 3) diagnostic or therapeutic agents loaded within the nanocarrier core or incorporated into the nanocarrier such that they are displayed on the nanocarrier surface. In order to form the preferred spherical nanoparticles, unsaturated sterol ester is also combined therewith. Diverse targeting may be achieved by conjugating certain targeting ligands, such as tumor-homing molecules (e.g., folic acid), to conjugable amino acids such as Lys residues exposed on the peptide's surfaces. The HPPS nanoparticles are thus directed to the desired cell surface markers of cancers and/or specific tissues, *i.e.,* molecules that are selectively overexpressed in various types of cancer cells or tissues. In particular embodiments, the HPPS nanoparticles provide targeted delivery of active agents including, but not limited to, diagnostic, imaging and/or therapeutic agents.

Such agents include, but are not limited to, magnetic resonance imaging (MRI) agents, near-infrared fluorescence (NIRF) probes and photodynamic therapy (PDT) agents.

In particular embodiments, the cholesterol esters inside the HPPS lipid core are replaced with lipophilic agents. In additional embodiments, active agents are attached to the surface of the HPPS nanoparticles of the present invention.

Thus, accumulation of the MRI/NIRF probes in target cells provides a facile mechanism for amplification of the MRI/NIRF detectable signal and affords opportunities to combine the strength of both MRI (high resolution/anatomic) and NIRF (high sensitivity), whereas the selective delivery of PDT agents to tumors via these pathways also provides a facile transition between detection and treatment. Finally, HPPS nanoparticles are not expected to be immunogenic and should escape recognition by the reticuloendothelial system, thus the HPPS platform provides a solution to common problems associated with most synthetic nanodevices, namely biocompatibility and toxicity.

The terms "nanoparticles", "nanoplatforms" and "nanoscaffold" are used interchangeably herein.

The invention provides HPPS nanoparticles comprising lipids such as phosphatidylcholine (eg. DMPC (preferably 1,2-Dimyristoyl-sn-Glycero-3-Phosphocholine), POPC (1-palmitoyl-1-oleoyl-phosphatidylcholine) and EYPC (egg yolk phosphatidylcholine)), lysophosphatidylcholine, phosphatidyl-ethanolamine, phosphatidylserine, and phosphatidylinositol.

The naturally occurring lipoprotein particles each have characteristic apoproteins, and percentages of protein, triacylglycerol, phospholipids and cholesterol. VLDL particles can contain about 10% protein, about 60% triacylglycerols, about 18% phopholipids and about 15% cholesterol. LDL particles can contain about 25% protein, about 10% triacylglycerols, about 22% phopholipids and about 45% cholesterol. HDL particles can contain about 50% protein, about 3% triacylglycerols, about 30% phopholipids and about 18% cholesterol. Likewise, the HPPS nanoparticles of the invention contain different percentages of the above constituents. The HPPS nanoparticles may also contain one or more triacylglycerols. The HPPS nanoparticles may additionally contain a sterol, a sterol ester, or combinations thereof. In other embodiments, the sterol or sterol ester is selected from the group consisting of a cholesterol, cholesterol oleate and an unsaturated cholesterol-ester.

In particular embodiments, the HPPS nanoparticles of the present invention contain cell surface receptor ligands which target receptors that are over-expressed in cancer cells. In other embodiments, the HPPS nanoparticles of the present invention contain cell surface receptor ligands that are ligands for a receptor over-expressed in cardiovascular plaques.

In additional embodiments, the HPPS nanoparticles of the present invention contain cell surface receptor ligands that target receptors on specific tissue(s).

In certain embodiments, the HPPS nanoparticles of the present invention contain lipophilic compounds in the core of the HPPS nanoparticles.

In certain embodiments, the HPPS nanoparticles of the present invention contain active agents that are molecules comprising a lipophilic and a hydrophilic component that are located (in part) on the surface of the HPPS nanoparticles.

In particular embodiments, the HPPS nanoparticles of the present invention contain an active agent that is a diagnostic agent, imaging agent or a therapeutic agent. Preferably, the imaging or diagnostic agent is a contrast agent, a radioactive label and/or a fluorescent label.

In certain embodiments, the HPPS nanoparticles of the present invention contain anticancer agents. Such active agents can be a chemotherapeutic agent, a photodynamic therapy agent, a boron neutron capture therapy agent or a radionuclide for radiation therapy. In embodiments, the anticancer agent is selected from the group consisting of alkylators, anthracyclines, antibiotics, aromatase inhibitors, bisphosphonates, cyclo-oxygnase inhibitors, estrogen receptor modulators, folate antagonists, inorganic arsenates, microtubule inhibitors, modifiers, nitrosureas, nucleoside analogs, osteoclast inhibitors, platinum containing compounds, retinoids, topoisomerase 1 inhibitors, kinase inhibitors (such as, without limitation, tyrosine kinase inhibitors), antiangiogenesis agents, epidermal growth factor inhibitors and histone deacetylase inhibitors.

In additional embodiments, the HPPS nanoparticles of the present invention contain a therapeutic agent selected from the group consisting of an antiglaucoma drug, an anti-clotting agent, an anti-inflammatory drug, an anti-asthmatic, an antibiotic, an antifungal or an antiviral drug.

In additional embodiments, the HPPS nanoparticles of the invention contain a therapeutic agent selected from the group including, but not limited to, analgesic agents, anesthetic agents, anti-anginal agents, antiarthritic agents, anti-arrhythmic agents, antiasthmatic agents, antibacterial agents, anti-BPH agents, anticancer agents, anticholinergic agents, anticoagulants, anticonvulsants, antidepressants, antidiabetic agents, antidiarrheals, anti-epileptic agents, antifungal agents, antigout agents, antihelminthic agents, antihistamines, antihypertensive agents, antiinflammatory agents, antimalarial agents, antimigraine agents, antimuscarinic agents, antinauseants, antineoplastic agents, anti-obesity agents, antiosteoporosis agents, antiparkinsonism agents, antiprotozoal agents, antipruritics, antipsychotic agents, antipyretics, antispasmodics, antithyroid agents, antitubercular agents, anti-tussives, antiulcer agents, anti-urinary incontinence agents, antiviral agents, anxiolytics, appetite suppressants, attention deficit disorder (ADD) and attention deficit hyperactivity disorder (ADHD) drugs, calcium channel blockers, cardiac inotropic agents, beta-blockers, cell adhesion inhibitors central nervous system stimulants, cognition enhancers, corticosteroids, COX-2 inhibitors, cytokine receptor activity modulators, decongestants, diuretics, erectile dysfunction improvement agents, essential fatty acids, gastrointestinal agents, genetic materials, histamine receptor antagonists, hormonolytics, hypnotics, hypoglycemic agents, immunosuppressants, keratolytics, leukotriene inhibitors, lipid-regulating agents, macrolides, mitotic inhibitors, muscle relaxants, narcotic antagonists, neuroleptic agents, nicotine, nitrates, non-essential fatty acids, non-steroid anti-asthma agents, nutritional oils, opioid analgesics, parasympatholytic agents, sedatives, sex hormones, stimulants, sympathomimetic agents, tranquilizers, vasodilators, vitamins, and combinations thereof.

The invention also provides pharmaceutical formulations comprising the HPPS nanoparticles of the present invention.

The invention further provides methods of making the HPPS nanoparticles.

### LIPOPROTEIN PARTICLES

Lipoprotein particles are a class of naturally occurring nanostructures. Cholesterol and triacylglycerols are transported in body fluids in the form of lipoprotein particles. Each particle consists of a core of hydrophobic lipids surrounded by a shell of more polar lipids and proteins. The protein components of these macromolecular aggregates have two roles: they solubilize hydrophobic lipids and contain cell-targeting signals. Lipoprotein particles are classified according to increasing density: chylomicrons, chylomicron remnants, very low density lipoprotein (VLDL), intermediate-density lipoproteins (IDL), low-density lipoprotein (LDL), and high density lipoproteins (HDL) (See Table 1). Accordingly, each of them is different in size, and most of them have nanostructures (<100 nm) with the exception of chylomicrons and chylomicron remnants.

**Table 1. Size and class of lipoproteins**

| Lipoproteins | Major Core Lipids | Apoproteins | Size |
|---|---|---|---|
| Chylomicrons | Dietary triacylglycerols, cholesterol esters | B-48, C, E | 75-1200 nm |
| VLDL | Endogenous triacylglycerols | B-100, C, E | 30-80 nm |
| IDL | Endogenous cholesterol esters | B-100, E | 25-35 nm |
| LDL | Endogenous cholesterol esters | B-100 | 18-25 nm |
| HDL | Endogenous cholesterol esters | A, C, E | 8-12 nm |

### Low Density Lipoprotein (LDL) Particles

LDL is the principal carrier of cholesterol in human plasma and delivers exogenous cholesterol to cells by endocytosis via the LDLR (Low Density Lipoprotein Receptor). The LDL particle is a naturally occurring nanostructure typically with a diameter of ∼22 nm. It contains a lipid core of some 1500 esterified cholesterol molecules and triglycerides. A shell of phospholipids and unesterified cholesterol surrounds this highly hydrophobic core. The shell also contains a single copy of apoB-100 (molecular weight 550kDa) which is recognized by the LDLR.

### High Density Lipoprotein (HDL) Particles

Plasma HDL is a small, spherical, dense lipid-protein complex that is approximately half lipid and half protein. The lipid component consists of phospholipids, free cholesterol, cholesteryl esters, and triglycerides. The protein component includes apo A-I (molecular weight, 28,000 Daltons) and apo A-II (molecular weight, 17,000 Daltons). Other minor but important proteins are apo E and apo C, including apo C-I, apo C-II, and apo C-III.

HDL particles are heterogeneous. They can be classified as a larger, less dense HDL2 or a smaller, more dense HDL3. Normally, most of the plasma HDL is found in HDL3. HDL is composed of 4 apolipoproteins per particle. HDL may be composed of both apo A-I and apo A-II or of apo A-I only. HDL2 is predominantly apo A-I only, and HDL3 is made of both apo A-I and apo A-II. HDL particles that are less dense than HDL2 are rich in apo E.

### HPPS Nanoparticles

Accordingly, the present invention provides a series of nanoplatforms with different sizes that can be made from a variety of different peptide scaffolds. Peptide components suitable for the production of HPPS nanoparticles are described below.

The HPPS nanoparticles can be targeted to various receptors. Moreover, in certain embodiments, both the HPPS hydrophobic core and phospholipids monolayer can be modified to carry large payloads of diagnostic and/or therapeutic agents making them exceptional multifunctional nanoplatforms. In certain embodiments, the HPPS nanoparticles of the present invention contain one or more homing molecules. The HPPS nanoparticles also can carry payloads of one or more active agents. The HPPS nanoparticles can also contain a cell death sensor so such HPPS nanoparticles can simultaneously perform diagnosis, treatment as well as therapeutic response monitoring functions.

The invention provides non-naturally occuring nano-platforms that are based on peptides having particular properties as described below. The term "non-naturally occurring" refers to nanoplatforms that do not exist innately in the human body. Such non-naturally occuring HPPS nanoparticles can contain one or more components of naturally occurring lipoprotein particles. For example, cholesterol esters that exist in the core of naturally occurring LDL and HDL particles may be replaced with active agents. Likewise, the core as found in the naturally occurring lipoprotein particles can remain intact, but an active agent is attached to the surface of the HPPS nanoparticles.

In some embodiments, there are several distinct advantages for using HPPS nanoparticles for targeted delivery. In some embodiments, one advantage of the HPPS nanoparticles of the present invention is that they are expected to be completely compatible with the host immune system, and they are also biodegradable. They can also provide a recycling system for accumulation of large quantities of diagnostic or therapeutic agents for target cells that utilize receptor mediated endocytosis. The HPPS nanoparticles of the present invention are not expected to be immunogenic and should escape recognition by the reticuloendothelial system (RES).

Other advantages can include: 1) the HPPS nanoparticles of the present invention bear resemblance to physiological carriers in that they have an ideal size range which is large enough to avoid renal clearance but small enough to mitigate reticuloendothelial system (RES) uptake, thus the serum half-life of drugs/probes may be prolonged by incorporation into these nanoparticles; 2) drug/probe sequestration in the lipid core space provides protection from serum enzyme and water; 3) the availability of the array of HPPS nanoparticles provides a series of nanoplatforms with size ranging from 5 nm to 80 nm.

Preferably, the non-naturally occurring HPPS nanoparticles of the present invention are (in increasing preferability) from 5 nm to 50 nm in diameter, from 5 nm to 40 nm in diameter; from 5 nm to 30 nm in diameter, from 5 nm to 25 nm in diameter; from 5 nm to 20 nm in diameter, and from 10 nm to 15 nm in diameter.

The starting materials of the HPPS nanoparticles also contain at least one lipid that is on, for example, the outer layer of the particle. Lipids useful in the HPPS nanoparticles of the present invention include, but are not limited to, amphipathic lipids. Phospholipids useful in the present invention include, but are not limited to phosphatidylcholine (preferably DMPC (1,2-Dimyristoyl-sn-Glycero-3-Phosphocholine, POPC (1-palmitoyl-1-oleoyl-phosphatidylcholine) and EYPC (egg yolk phosphatidylcholine)), lysophosphatidylcholine, phosphatidylethanolamine, phosphatidylserine, phosphatidylinositol and combinations thereof.

### SCAFFOLD PEPTIDES

Suitable scaffold peptides for use in forming the HPPS nanoparticles comprise an amino acid sequence capable of forming at least one amphipathic α-helix.

In preferred embodiments, the amphipathic α-helix or peptide ranges in length (in increasing preferability) from 6-30 amino acids, 8-28 amino acids, 10-24 amino acids, 11-22 amino acids, 4-21 amino acids, 16-20 amino acids and 18 amino acids.

Methods for detecting and characterizing protein domains with putative amphipathic helical structure are set forth in Segrest, J. P. et al. in PROTEINS: Structure, Function, and Genetics (1990) 8:103-117, the contents of which are incorporated herein by reference. Segrest *et al.* have identified seven different classes of amphipathic helices and have identified peptides/proteins associated with each class. Of the seven different classes there are four lipid-associating amphipathic helix classes (A, H, L, and M). Of these, Class A, the designated apolipoprotein class, possesses optimal properties for forming phospholipid-based particles.

Suitable scaffold peptides may be selected from the group consisting of Class A, H, L and M α-helices or a fragment thereof. Suitable scaffold peptides may also comprise a reversed peptide sequence of the Class A, H, L and M amphipathic α-helices or a fragment thereof, as the property of forming an amphipathic α-helix is determined by the relative position of the amino acid residues within the peptide sequence.

In one embodiment, the scaffold peptide has an amino acid sequence comprising consecutive amino acids of an apoprotein, preferably selected from the group consisting of apoB-100, apoB-48, apoC, apoE and apoA.

The "amino acids" used in this invention, and the term as used in the specification and claims, include the known naturally occurring protein amino acids, which are referred to by both their common three letter abbreviation and single letter abbreviation. See generally Synthetic Peptides: A User's Guide, G A Grant, editor, W.H. Freeman & Co., New York, 1992, the teachings of which are incorporated herein by reference, including the text and table set forth at pages 11 through 24. As set forth above, the term "amino acid" also includes stereoisomers and modifications of naturally occurring protein amino acids, non-protein amino acids, post-translationally modified amino acids, enzymatically synthesized amino acids, derivatized amino acids, constructs or structures designed to mimic amino acids, and the like. Modified and unusual amino acids are described generally in Synthetic Peptides: A User's Guide, cited above; Hruby V J, Al-obeidi F and Kazmierski W: Biochem J 268:249-262,1990; and Toniolo C: Int J Peptide Protein Res 35:287-300,1990; the teachings of all of which are incorporated herein by reference.

"Alpha-helix" is used herein to refer to the common motif in the secondary structure of proteins. The alpha helix (a-helix) is a coiled conformation, resembling a spring, in which every backbone N-H group donates a hydrogen bond to the backbone C=O group of the amino acid four residues earlier. Typically, alpha helices made from naturally occurring amino acids will be right handed but left handed conformations are also known.

"Amphipathic" is a term describing a chemical compound possessing both hydrophilic and hydrophobic properties. An amphipathic alpha helix is an often-encountered secondary structural motif in biologically active peptides and proteins and refers to an alpha helix with opposing polar and nonpolar faces oriented along the long axis of the helix.

Examples of small amphipathic helix peptides, possessing many of the lipid binding properties of apoA-I include 2F, an 18-amino acid peptide sequence with two phenylalanine amino acid residues (D-W-L-K-A-F-Y-D-K-V-A-E-K-L-K-E-A-F) (Ananthaaramaiah et al. JBC. 1985; 260:10248-10255). Early studies showed that this sequence displayed strong lipid association and was able to form stable discoidal phospholipid particles. Subsequent studies went on to show that the incorporation of two additional F residues (4F total; D-W-F-K-A-F-Y-D-K-V-A-E-K-F-K-E-A-F), further enhanced the lipid associating properties of the peptide (Ananthaaramaiah et al. Arterioscler Thromb Vasc Biol. 2005; 25:1325-1331).

### HOMING MOLECULES

As used herein, the term "home" or "selectively home" means that a particular molecule binds relatively specifically to molecules present in specific organs or tissues following administration to a subject. In general, selective homing is characterized, in part, by detecting at least a two-fold greater selective binding of the molecule to an organ or tissue as compared to a control organ or tissue. In certain embodiments the selective binding is at least three-fold or four-fold greater as compared to a control organ or tissue.

In the case of tumor homing molecules, such molecules bind to receptors that are selectively over-expressed in particular cancer tissues. By over expression is meant at least one and one half greater expression in tumor tissue compared to normal tissue. In embodiments, expression is at least five times greater in tumor as compared to non-tumor.

In embodiments of the present invention, a homing molecule is attached to a peptide of the HPPS nanoparticle of the present invention that targets specific tissues and tumors. A "homing molecule" refers to any material or substance that may promote targeting of tissues and/or receptors *in vitro* or *in vivo* with the compositions of the present invention. The targeting moiety may be synthetic, semi-synthetic, or naturally-occurring. The targeting moiety may be a protein, peptide, oligonucleotide, or other organic molecule. In one embodiment, the targeting moiety may be an endogenous ligand (or part thereof) to a cell surface receptor. The targeting moiety may be an antibody (this term including antibody fragments and single chain antibodies that retain a binding region or hypervariable region). Materials or substances which may serve as targeting moieties include, but are not limited to, those substances listed in Table 2:

**Table 2**

| Targeting Moiety | Target(s) |
|---|---|
| Antibodies (and fragments such as Fab, F(ab)'2, Fv, Fc, etc.) | RES system |
| Epidermal growth factor (EGF) | Cellular receptors |
| Collagen | Cellular receptors |
| Gelatin | Cellular receptors |
| Fibrin-binding protein | Fibrin |
| Plasminogen activator | Thrombus |
| Urokinase inhibitor | Invasive cells |
| Somatostatin analogs | Cellular receptors |
| Lectin (WGA) | Axons |
| f-Met-Leu-Phe | Neutrophils |
| Selectin active fragments | Glycosyl structures |
| ELAM, GMP 140 | Leucocyte receptors |
| "RGD" proteins | Integrins, Granulocytes |
| IL-2 | Activated T-cell |
| CD4 | HIV infected cells |
| Cationized albumin | Fibroblasts |
| Carnitine | Mitochondria |
| Acetyl-, maleyl-proteins | Macrophage scavenger receptor |
| Hyaluronic acid | Cellular receptors |
| Lactosylceramide | Hepatocytes |
| Asialofoetuin | Hepatocytes |
| Arabinogalactan | Hepatocytes |
| Galactosylated particles | Kupffer cells |
| Terminal fucose | Kupffer cells |
| Mannose | Kupffer cells, macrophages |
| Lactose | Hepatocytes |
| Dimuramyl-tripeptide | Kupffer cells, macrophages |
| Fucoidin-dextran sulfate | Kupffer cells, macrophages |
| Sulfatides | Brain |
| Glycosyl-steroids | |
| Glycosphyngolipids | Other glycosylated structures |
| Hypoxia mediators | Infarcted tissues |
| Amphetamines | Nervous system |
| Barbiturates | Nervous system |
| Sulfonamides | |
| Monoamine oxidase inhibitor substrates | Brain |
| Chemotactic peptides | Inflammation sites |
| Muscarine and dopamine receptor substrates | Nervous system |

### Tumor Homing Molecules

Tumor homing molecules selectively bind to tumor tissue versus normal tissue of the same type. Such molecules in general are ligands for cell surface receptors that are over-expressed in tumor tissue. Cell surface receptors over-expressed in cancer tissue versus normal tissue include, but are not limited to, epidermal growth factor receptor (EGFR) overexpressed in anaplastic thyroid cancer, colorectal cancer, head and neck cancer, ovarian cancer, renal cell cancer, and breast and lung tumors, metastin receptor overexpressed in papillary thyroid cancer, ErbB family receptor tyrosine kinases overexpressed in a significant subset of breast cancers, human epidemal growth factor receptor-2 (Her2/neu) overexpressed in breast cancers, tyrosine kinase receptor (c-Kit) overexpressed in sarcomatoid renal carcinomas, HGF receptor c-Met overexpressed in esophageal adenocarcinoma, CXCR4 and CCR7 overexpressed in breast cancer, endothelin-A receptor overexpressed in prostate cancer, peroxisome proliferator activated receptor delta (PPAR-delta) overexpressed in most colorectal cancer tumors, PDGFR A overexpressed in ovarian carcinomas, BAG-1 overexpressed in various lung cancers, soluble type II TGF beta receptor overexpressed in pancreatic cancer, folate, and integrin (e.g. *α*v*β*₃).

The folate receptor is a glycosylphosphatidylinositol-anchored glycoprotein with high affinity for the vitamin folic acid (Kd ∼ 10⁻⁹ M) (Leamon, C.P. et al., Biochemical Journal. 1993 May 1; 291 (Pt. 3):855-60). Folate receptor has been identified as a tumor-marker, which is expressed at elevated levels relative to normal tissues on epithelial malignancies, such as ovarian, colorectal, and breast cancer (Wang, S. et al., Journal of Controlled Release. 1998 Apr 30;53(1-3):39-48). It has been shown that when folate is covalently linked to either a single molecule or assembly of molecules via its g-carboxyl moiety, its affinity for the cell surface receptors remains essentially unaltered. Following endocytosis and vesicular trafficking, much of the material is released into the cell cytoplasm. The unligated folate receptor may then recycle to the cell surface; thus, each folate receptor may bring many folate conjugates into the cell.

### Organ or Tissue Homing Molecules

The present invention provides for the use of molecules that selectively home to various organs or tissues, including to lung, skin, blood, pancreas, retina, prostate, ovary, lymph node, adrenal gland, liver, breast, digestive system or renal tissue. For example, the invention provides using lung homing peptides such as those containing a GFE motif, including the peptides CGFECVRQCPERC and CGFELETC; skin homing peptides such as CVALCREACGEGC; pancreas homing peptides such as the peptide SWCEPGWCR; and retina homing peptides such as those containing an RDV motif, including the peptides CSCFRDVCC and CRDVVSVIC.

The invention also provides methods of using an organ homing molecule of the invention to diagnose or treat a pathology of the lung, skin, pancreas, retina, prostate, ovary, lymph node, adrenal gland, liver or gut by administering a HPPS comprising a molecule that homes to the selected organ or tissue of a subject having or suspected of having a pathology. For example, a pathology of lung, skin, pancreas, retina, prostate, ovary, lymph node, adrenal gland, liver or gut can be treated by administering to a subject having the pathology a HPPS nanoparticle comprising an appropriate organ homing molecule linked to a therapeutic agent. Similarly, a method of identifying a selected organ or tissue or diagnosing a pathology in a selected organ by administering to a subject a HPPS nanoparticle comprising an appropriate organ homing molecule linked to a detectable agent.

The HPPS nanoparticles of the present invention can be used with organ and tissue homing molecules to target a moiety to a selected organ or tissue. The homing molecules employed in the invention include peptides that home to various normal organs or tissues, including lung, skin, pancreas, retina, prostate, ovary, lymph node, adrenal gland, liver or gut, and to organs bearing tumors, including to lung bearing lung tumors and to pancreas bearing a pancreatic tumor. For example, the invention includes the use of lung homing peptides, including the peptides CGFECVRQCPERC and CGFELETC, each of which contains a tripeptide GFE motif, and the peptide GIGEVEVC. The invention also includes the use of skin homing peptides such as the peptide CVALCREACGEGC; pancreas homing peptides such as the peptide SWCEPGWCR and retina homing peptides such as the peptides CSCFRDVCC and CRDVVSVIC, each of which contains a tripeptide RDV motif. Examples of peptides that home to prostate, ovary, lymph node, adrenal gland, liver and gut are also provided (see Table 3 below). In one embodiment, the homing molecule may be EGFR-specific peptide such as full-length EGF protein (EGF) or a fragment thereof.

For convenience, the term "peptide" is used broadly herein to mean peptides, polypeptides, proteins and fragments of proteins and includes, for example, single-chain peptides. Other molecules useful in the invention include peptoids, peptidomimetics and the like. With respect to the organ or tissue homing peptides of the invention, peptidomimetics, which include chemically modified peptides, peptide-like molecules containing nonnaturally occurring amino acids, peptoids and the like, have the binding activity of an organ homing peptide upon which the peptidomimetic is derived (see, for example, "Burger's Medicinal Chemistry and Drug Discovery" 5th ed., vols. 1 to 3 (ed. M. E. Wolff; Wiley Interscience 1995), which is incorporated herein by reference). Peptidomimetics provide various advantages over a peptide, including that a peptidomimetic can be stable when administered to a subject, for example, during passage through the digestive tract and, therefore, useful for oral administration.

Methods for identifying a peptidomimetic are well known in the art and include, for example, the screening of databases that contain libraries of potential peptidomimetics. For example, the Cambridge Structural Database contains a collection of greater than 300,000 compounds that have known crystal structures (Allen et al., Acta Crystallogr. Section B, 35:2331 (1979)). This structural depository is continually updated as new crystal structures are determined and can be screened for compounds having suitable shapes, for example, the same shape as an organ or tissue homing molecule, as well as potential geometrical and chemical complementarity to a target molecule bound by an organ or tissue homing peptide. Where no crystal structure of a homing peptide or a target molecule, which binds an organ or tissue homing molecule, is available, a structure can be generated using, for example, the program CONCORD (Rusinko et al., J. Chem. Inf. Comput. Sci. 29:251 (1989)). Another database, the Available Chemicals Directory (Molecular Design Limited, Informations Systems; San Leandro Calif.), contains about 100,000 compounds that are commercially available and also can be searched to identify potential peptidomimetics of an organ or tissue homing molecule.

Selective homing of a molecule to a selected organ or tissue can be due to selective recognition by the molecule of a particular cell target molecule such as a cell surface protein present on a cell in the organ or tissue. Selectivity of homing is dependent on the particular target molecule being expressed on only one or a few different cell types, such that the molecule homes to only one or a few organs or tissues. In this regard, most different cell types, particularly cell types that are unique to an organ or tissue, can express unique target molecules.

Examples of peptide motifs that have been identified as useful for homing to particular organs or tissue include those listed in Table 3.

**Table 3**

| ORGAN/MOTIF | |
|---|---|
| GUT | PROSTATE |
| YSGKWGK | SMSIARL |
| GISALVLS | VSFLEYR |
| SRRQPLS | RGRWLAL |
| MSPQLAT | |
| VRRGSPQ | |
| MRRDEQR | |
| QVRRVPE | |
| GGRGSWE | |
| FRVRGSP | |
| RVRGPER | |
| LIVER | ADRENAL GLAND |
| VKSVCRT | LMLPRAD |
| WRQNMPL | LPRYLLS |
| SRRFVGG | |
| ALERRSL | |
| ARRGWTL | |
| PANCREAS | OVARY |
| SWCEPGWCR | EVRSRLS |
| | VRARLMS |
| | RVGLVAR |
| | RVRLVNL |
| SKIN | LUNG |
| CVALCREACGEGC | CTLRDRNC |
| CSSGCSKNCLEMC | CGKRYRNC |
| CIGEVEVC | CLRPYLNC |
| CKWSRLHSC | CGFELETC |
| CWRGDRKIC | CTVNEAYKTRMC |
| CERVVGSSC | CRLRSYGTLSLC |
| CLAKENVVC | CRPWHNQAHTEC |
| | CGFECVRQCPERC |

The invention includes the use of lung homing peptides such as CGFECVRQCPERC and CGFELETC, which share a GFE motif; CTLRDRNC; and CIGEVEVC which contains an EVE motif that is similar to the ELE motif present in CGFELETC.

Preferably, the invention also can use skin homing peptides such as CVALCREACGEGC. The invention further provides HPPS nanoparticles with pancreas homing peptides such as SWCEPGWCR. Retina homing peptides such as CSCFRDVCC and CRDVVSVIC can also be used in conjuction with the HPPS nanoparticles of the present invention. Prostate homing peptides such as SMSIARL and VSFLEYR, can also be used with the HPPS nanoparticles of the present invention. Also provided are ovary homing peptides such as RVGLVAR and EVRSRLS. The invention also can use adrenal gland homing peptides such as LMLPRAD and LPRYLLS, which share a LPR motif or the peptides R(Y/F)LLAGG and RYPLAGG, which share the motif LAGG. In addition, lymph node homing peptides, such as AGCSVTVCG can be used in conjunction with the present invention. The invention also can use gut homing peptides such as YSGKWGK and YSGKWGW.

### Cardiovascular Plaque Homing Molecules

Atherosclerosis plaques are known to over-express certain receptors, such as CX3CL1. The invention therefore includes ligands for receptors over-expressed on such plaques.

### Infected Tissue Homing Molecules

Tissue infected with a variety of infective agents such as viruses, parasites, and/or bacteria (e.g. HIV, malaria...etc.), typically express (or over-express) cell surface markers/receptors (e.g. proteins). Homing molecules of the invention therefore include ligands for said cell surface markers/receptors expressed on such infected tissue.

### ACTIVE AGENTS

Methods of modifying and packaging active agents into LDL are described by Krieger, M. in Methods Enzymol. (1986)128:608-13, the contents of which are herein incorporated by reference in this regard. Similar methods may be used in conjunction with the present invention.

### Lipophilic Compounds

A variety of active agents can be delivered via the HPPS nanoparticles of the present invention. In embodiments, the active agent is located in the core of the HPPS nanoparticles, which is generally lipophilic. Lipophilic compounds are therefore able to be delivered via the HPPS nanoparticles of the present invention. The HPPS nanoparticles of the present invention can be used with active agents that are inherently lipophilic or can be made lipophilic by chemical modification, discussed in more detail below.

The term "lipophilic compound" or "lipophilic drug" is defined as a compound or drug which in its non-ionized form is more soluble in lipid or fat than in water. Examples of lipophilic compounds include, but are not limited to, acetanilides, anilides, aminoquinolines, benzhydryl compounds, benzodiazepines, benzofurans, cannabinoids, cyclic peptides, dibenzazepines, digitalis gylcosides, ergot alkaloids, flavonoids, imidazoles, quinolines, macrolides, naphthalenes, opiates (or morphinans), oxazines, oxazoles, phenylalkylamines, piperidines, polycyclic aromatic hydrocarbons, pyrrolidines, pyrrolidinones, stilbenes, sulfonylureas, sulfones, triazoles, tropanes, and vinca alkaloids.

A variety of tests can be used to determine lipophilicity. A common test protocol is measurement of the octanol-water partition coefficient (P_{OW}, K_{OW}), which is a measure of lipophilicity by determination of the equilibrium distribution between octan-1-ol and water. Lipophilic drugs are those drugs that preferably partition into the octanol component.

Pharmaceutically active lipophilic drugs which may be incorporated into targeted drug delivery complexes of the invention include drugs for the treatment of cancer and glaucoma, immunoactive agents, antineoplastic agents, anticholinergic and cholinomimetic agents, antimuscarinic and muscarinic agents, antiadrenergic and antiarrhythmics, antihypertensive agents, anti-inflammatory drugs, antibiotic drugs, anti-fungal drugs, steroids, anti-histamines, anti-asthmatics, sedatives, anti-epileptics, anesthetics, hypnotics, antipsychotic agents, neuroleptic agents, antidepressants, anxiolytics, anti-convulsant agents, neuron blocking agents, narcotic antagonists, analgesics, anti-proliferative agents, anti-viral drugs, hormones, and nutrients.

Examples of anti-cancer drugs include but are not limited to paclitaxel, docosahexaenoic acid (DHA)-paclitaxel conjugates, cyclophosphoramide, betulinic acid, and doxorubicin (see, e.g. U.S. Pat. No. 6,197,809 to Strelchenok).

Examples of anti-glaucoma drugs include but are not limited to *β*-blockers such as timolol-base, betaxolol, atenolol, livobunolol, epinephrine, dipivalyl, oxonolol, acetazolamide-base and methzolamide.

Examples of anti-inflammatory drugs include but are not limited to steroidal drugs such as cortisone and dexamethasone and non-steroidal anti-inflammatory drugs (NSAID) such as piroxicam, indomethacin, naproxen, phenylbutazone, ibuprofen and diclofenac acid. Examples of anti-asthmatics include but are not limited to prednisolone and prednisone. (See also U.S. Pat. No. 6,057,347).

An example of an antibiotic drug includes but is not limited to chloramphenicol. Examples of anti-fungal drugs include but are not limited to nystatin, amphotericin B, and miconazole. Examples of an anti-viral drug includes but is not limited to Acyclovir™ (Glaxo Wellcome, U.K.).

Examples of steroids include but are not limited to testosterone, estrogen, and progesterone. Examples of anti-allergic drugs include but are not limited to pheniramide derivatives. Examples of sedatives include but is not limited to diazepam and propofol.

Compounds to be delivered that are not ordinarily lipophilic may be used in the present invention by fusing or covalently coupling them to one or more lipophilic molecule(s) to produce an amphiphathic compound. Such lipophilic molecules preferably contain at least one long hydrocarbon chain (>C₁₀) which is either bent by a cis-double bond or branched by at least one side chain, for example. Such molecules include, but are not limited to, cholesterol oleate, oleate, cholesterol laurate or phytol. Sterols and fatty acids can also be used.

Nucleic acids, such as siRNA, and both nucleic acid and peptide aptamers, may be delivered as "lipophilic" compounds by complexing the nucleic acid with a cationic lipid to form a lipophilic complex which can then be incorporated into the core of the particle, or otherwise associated with the phospholipids of the invention. Nucleic acids may also be incorporated into the HPPS nanoparticles *via* covalent linkage to lipid anchors, as noted below.

### Agents with Lipid Anchors

In addition to active agents that are lipophilic and can be loaded into the core of the HPPS nanoparticles of the present invention, the invention also includes active agents that can be loaded onto the surface of the HPPS of the present invention. Such active agents can be hydrophilic with a lipid anchor. The HPPS nanoparticles of the present invention can also be modified to include a lipophilic chelator, such lipophilic chelators are well known. For example, the lipophilic chelator, DTPA Bis (stearylamine), can be incorporated into the HPPS nanoparticle using standard techniques. Likewise 1,1-dioctadecyl-3,3,3,3-tetramethylindocarbocyanine perchlorate (DiI), can be used as a lipid-anchored, carbocanine based optical probe known to intercalate into the LDL phospholipid monolayer and can be used in the HPPS nanoparticles of the present invention

Similarly, near infrared ("NIR") probes such as tricarbocyanine dyes, which are NIR fluorophores, can be modified to include a lipid-chelating anchor that allows such probes to be anchored to the HPPS nanoparticles of the present invention. Any such lipid-chelating anchors can be used, for example, a cholesteryl laurate moiety can be attached to the NIR probes to anchor them to the HPPS nanoparticles of the present invention. (Zheng et al., Bioorg. & Med. Chem Lett. 12:1485-1488 (2002).

As noted above, cholesterol moieties may also be used to anchor siRNA (and additionally aptamers) and other active agents within the core of the HPPS nanoparticles. It has been established that cholesterol-conjugated siRNAs can silence gene expression in vivo and that these conjugates bind to LDL and HDL particles (Wolfrum, C. et al. Nature Biotech. published online 16 September 2007). Suitable anchors for active agents include oleate and unsaturated cholesterol ester moieties. Such anchors may be covalently bound to active agents using synthetic methods known to those of skill in the art. In one embodiment, the anchors may be covalently bound to the active agent *via* an ester linkage.

### Imaging/Diagnostic Agents

In one embodiment, an active agent can be a detectable agent such as a radionuclide or an imaging agent, which allows detection or visualization of the selected organ or tissue. Thus, the invention provides a HPPS nanoparticle comprising a lung, skin, blood, pancreas, retina, prostate, ovary, lymph node, adrenal gland, liver or gut homing molecule. The type of detectable agent selected will depend upon the application. For example, for an in vivo diagnostic imaging study of the lung in a subject, a lung homing molecule can be linked to a HPPS nanoparticle comprising an agent that, upon administration to the subject, is detectable external to the subject. For detection of such internal organs or tissues, for example, the prostate, a gamma ray emitting radionuclide such as indium-113, indium-115 or technetium-99 can be conjugated with a HPPS nanoparticle that is linked to a prostate homing molecule and, following administration to a subject, can be visualized using a solid scintillation detector. Alternatively, for organs or tissues at or near the external surface of a subject, for example, retina, a fluorescein-labeled retina homing molecule can be used such that the endothelial structure of the retina can be visualized using an opthalamoscope and the appropriate optical system.

Molecules that selectively home to a pathological lesion in an organ or tissue similarly can be used in the HPPS nanoparticle of the invention to deliver an appropriate detectable agent such that the size and distribution of the lesion can be visualized. For example, where an organ or tissue homing molecule homes to a normal organ or tissue, but not to a pathological lesion in the organ or tissue, the presence of the pathological lesion can be detected by identifying an abnormal or atypical image of the organ or tissue, for example, the absence of the detectable agent in the region of the lesion.

A detectable agent also can be an agent that facilitates detection in vitro. For example, a HPPS nanoparticle comprising a homing molecule and an enzyme, which produces a visible signal when an appropriate substrate is present, can detect the presence of an organ or tissue or cells to which the homing molecule is directed. Such a HPPS nanoparticle, which can comprise, for example, alkaline phosphatase or luciferase or the like, can be useful in a method such as immunohistochemistry. Such a HPPS nanoparticle also can be used to detect the presence of a target molecule, to which the organ homing molecule binds, in a sample, for example, during purification of the target molecule.

Additional diagnostic agent include contrast agents, radioactive labels and fluorescent labels. Preferred contrast agent are optical contrast agents, MRI contrast agents, ultrasound contrast agents, X-ray contrast agents and radio-nuclides.

### Therapeutic Agents

A therapeutic agent can be any biologically useful agent that exerts its function at the site of the selected organ or tissue, such as the active agents and lipophilic compounds previously mentioned. For example, a therapeutic agent can be a small organic molecule that, upon binding to a target cell due to the linked organ homing molecule, is internalized by the cell where it can effect its function. A therapeutic agent can be a nucleic acid molecule that encodes a protein involved in stimulating or inhibiting cell survival, cell proliferation or cell death, as desired, in the selected organ or tissue. For example, a nucleic acid molecule encoding a protein such as Bcl-2, which inhibits apoptosis, can be used to promote cell survival, whereas a nucleic acid molecule encoding a protein such as Bax, which stimulates apoptosis, can be used to promote cell death of a target cell.

A particularly useful therapeutic agent that stimulates cell death is ricin, which, when linked to a HPPS comprising an organ homing molecule of the invention, can be useful for treating a hyperproliferative disorder, for example, cancer. A HPPS nanoparticle comprising an organ homing molecule of the invention and an antibiotic, such as ampicillin or an antiviral agent such as ribavirin, for example, can be useful for treating a bacterial or viral infection in a selected organ or tissue.

A therapeutic agent also can inhibit or promote the production or activity of a biological molecule, the expression or deficiency of which is associated with the pathology. Thus, a protease inhibitor can be a therapeutic agent that, when linked to a HPPS comprising an organ homing molecule, can inhibit protease activity at the selected organ or tissue, for example, the pancreas. A gene or functional equivalent thereof such as a cDNA, which can replenish or restore production of a protein in a selected organ or tissue, also can be a therapeutic agent useful for ameliorating the severity of a pathology. A therapeutic agent also can be an antisense nucleic acid molecule, the expression of which inhibits production of a deleterious protein, or can be a nucleic acid molecule encoding a dominant negative protein or a fragment thereof, which can inhibit the activity of a deleterious protein. As noted above, nucleic acids, and nucleic acid and peptide aptamers, may be delivered as "lipophilic" compounds by complexing the nucleic acid with a cationic lipid to form a lipophilic complex which can then be incorporated into the core of the particle, or otherwise associated with the phospholipids of the invention. Cholesterol moieties or other lipid anchors may also be used to anchor these and other active agents within the core of the HPPS nanoparticles.

### Photodynamic Therapy (PDT) Agents

PDT is a promising cancer treatment that involves the combination of light and a photosensitizer. Each factor is harmless by itself, but when combined together, they can produce lethal reactive oxygen species that kill the tumor cells (Dougherty, T.J. et al. Journal of the National Cancer Institute. 90, 889-905 (1998)). Singlet oxygen (¹O₂) is a powerful, fairly indiscriminate oxidant that reacts with a variety of biological molecules and assemblies. It is generally recognized that ¹O₂ is the key agent of PDT induced tumor necrosis (Niedre, M. et al. Photochemistry & Photobiology. 75, 382-391 (2002)). The diffusion range of ¹O₂ is limited to approximately 45 nm in cellular media (Moan, J. Photochem. Photobiol. 53, 549-553 (1991)). Therefore, the site of the primary generation of O₂ determines which subcellular structures may be accessed and attacked. In other words, if a photosensitizer is preferentially localized in tumor cells, PDT induced cellular damage is highly tumor specific.

Preferred photodynamic therapy agents are porphyrins, porphyrin isomers, and expanded porphyrins.

In embodiments, the photodynamic therapy agent is selected from the group consisting of tert-butyl silicon naphthalocyanine bisoleate (SiNc-BOA), tert-butyl silicon phthalocyanine bisoleate (SiPc-BOA), and pyropheophorbide-cholesterol ester (Pyro-CE). SiNc-BOA, SiPc-BOA, and Pyro-CE are photosensitizer compounds that produce toxic oxygen species for photodynamic therapy.

### Near-Infrared (NIR) Dyes for Fluorescent Imaging and PDT Agents

NIR fluorescent imaging (NIRF) is a non-radioactive, highly sensitive, and inexpensive cancer detection modality (Weissleder, R. et al. Nature Medicine 9, 123-128 (2003), Frangioni, J.V. Current Opinion in Chemical Biology 7, 626-634 (2003)), which permits noninvasive differentiation of tumor and healthy tissue based on differences in their fluorescence. NIR dyes are presently attracting considerable interest as NIRF probes for detection of cancer and as photosensitizers for cancer treatment by PDT. The appeal of NIR dyes resides in the tissue optical properties in the spectral window between 600 nm to 900 nm. For such wavelengths, the tissue absorption coefficients are relatively low; thus, the propagation of light is mainly governed by scattering events, and a penetration depth of several centimeters is attainable. Therefore, the unique capability of NIR dyes enables fluorescent imaging and PDT treatment of subsurface tumors, including breast cancer.

A current limitation of both NIRF and PDT modalities is their lack of sufficient tumor-to-tissue contrast due to the relatively non-specific nature of delivering the dye to the tumor, which has led to false negatives for NIRF and inadequate tumor-to-normal tissue therapeutic ratio for PDT. Hence, agents targeting "cancer signatures", i.e. molecules that accumulate selectively in cancer cells, are particularly attractive. This invention provides tumor-targeting HPPS nanoparticles locking NIRF/PDT agents inside the core so that a higher probe/protein molar ratio and tumor specificity is achieved.

### Magnetic Resonance Imaging Agents

It is now well established that MRI is the pre-eminent methodology among the various diagnostic modalities currently available, as it offers a powerful way to map structure and function in soft tissues by sampling the amount, flow, and environment of water protons in vivo. The intrinsic contrast can be augmented by the use of contrast agents. Targeted MRI agents, though extremely attractive conceptually, exist in only a few potentially useful examples. Because of sensitivity limitations, efficient recognition currently requires a very high capacity target like fibrin, which is present in sufficient quantity to be seen with simple targeted Gd (gadolinium) chelates, or targets accessible to the blood stream that can be bound with a Gd cluster, polymer or an iron particle. This is presently a very limited target set. Moreover, intracellular MRI imaging is particularly challenging because the minimum concentration of MRI agents required for the MRI detection limit is much higher (∼ 1 mM) than the extracellular targeting threshold (40 µ1V1) (Aime, S. et al. Journal of Magnetic Resonance Imaging. 2002 16(4):394-406, Nunn, A.D. et al. Quarterly Journal of Nuclear Medicine. 1997 41(2):155-62). One notable attempt was reported by Wiener *et al.* in 1995 (Wiener, E.C. et al. Investigative Radiology. 1997 Dec,-32(12):748-54). Using a folate-conjugated DTPA-based dendrimer, they achieved uptake by tumor cells which was related to the presence of the folate receptor. They also obtained MRI contrast enhancement of 17% 24 h after injection. The present invention provides HPPS nanoparticles to deliver both MRI and NIRF/PDT agents.

In certain embodiments of the present invention, the MRI contrast agent is an iron oxide or a lanthanide base, such as gadolinium (Gd³⁺) metal.

### Agents Active in the Nervous System

Drugs that are active on the nervous system can also be delivered via the HPPS nanoparticles of the present invention. Such drugs include antipsychotics, stimulants, sedatives, anesthetics, opiates, tranquilizers, antidepressants, such as MAO inhibitors, tricyclics and tetracyclics, selective serotonin reuptake inhibitor and burpropion. Drugs that are active in the nervous system also include neuropeptides. Delivery of peptide drugs is limited by their poor bioavailability to the brain due to low metabolic stability, high clearance by the liver and the presence of the blood brain barrier. The HPPS nanoparticles of the present invention enable the delivery of such drugs to the central nervous system.

### Pharmaceutical Compositions

When administered to a subject, the nanoplatforms of the present invention are administered as a pharmaceutical composition containing, for example, the HPPS nanoparticles and a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil or injectable organic esters.

A pharmaceutically acceptable carrier can contain physiologically acceptable compounds that act, for example, to stabilize or to increase the absorption of the complex. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know that the choice of a pharmaceutically acceptable carrier, including any physiologically acceptable compound therein, depends, for example, on the route of administration of the pharmaceutical composition. The pharmaceutical composition can also contain a HPPS nanoparticle further comprising a cancer therapeutic agent, or other active agent as desired, including lipophilic compounds, agents with lipid anchors, imaging/diagnostic agents, therapeutic agents, photodynamic therapy (PDT) agents, near-infrared (NIR) dyes for fluorescent imaging and PDT agents, magnetic resonance imaging agents, and agents active in the nervous system.

As noted above, the nanoplatforms of the present invention may be provided in a physiologically or pharmaceutically acceptable carrier, or may be provided in a lyophilized form for subsequent use. The compositions are optionally sterile when intended for parenteral administration or the like, but need not always be sterile when intended for some topical application. Any pharmaceutically acceptable carrier may be used, including but not limited to aqueous carriers. Aqueous carriers for parenteral injections include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

One skilled in the art would know that a pharmaceutical composition containing the HPPS nanoparticles of the present invention can be administered to a subject by various routes including, for example, orally or parenterally, such as intravenously. The pharmaceutical composition can be administered by injection or by intubation.

In performing a diagnostic, imaging or therapeutic method as disclosed herein, a therapeutically effective amount of a HPPS nanoparticle of the present invention must be administered to the subject. A "therapeutically effective amount" is the amount of the HPPS nanoparticle that produces a desired effect. An effective amount will depend, for example, on the active agent and on the intended use. For example, a lesser amount of a radiolabeled HPPS nanoparticle can be required for imaging as compared to the amount of the radiolabeled molecule administered for therapeutic purposes, where cell killing is desired. A therapeutically effective amount of a particular HPPS nanoparticle for a specific purpose can be determined using methods well known to those in the art.

In principle, an organ homing molecule as part of a HPPS nanoparticle of the present invention can have an inherent biological property, such that administration of the molecule provides direct biological effect. For example, an organ homing molecule can be sufficiently similar to a naturally occurring ligand for the target molecule that the organ homing molecule mimics the activity of the natural ligand. Such an organ homing molecule can be useful as a therapeutic agent having the activity of the natural ligand. For example, where the organ homing molecule mimics the activity of a growth factor that binds a receptor expressed by the selected organ or tissue, such as a skin homing molecule that mimics the activity of epidermal growth factor, administration of the organ homing molecule can result in cell proliferation in the organ or tissue. Such inherent biological activity of an organ homing molecule of the invention can be identified by contacting the cells of the selected organ or tissue with the homing molecule and examining the cells for evidence of a biological effect, for example, cell proliferation or, where the inherent activity is a toxic effect, cell death.

In addition, an organ homing molecule as part of the HPPS nanoparticle of the invention can have an inherent activity of binding a particular target molecule such that a corresponding ligand cannot bind the receptor. It is known, for example, that various types of cancer cells metastasize to specific organs or tissues, indicating that the cancer cells express a ligand that binds a target molecule in the organ to which it metastasizes. Thus, administration of a lung homing molecule, for example, to a subject having a tumor that metastasizes to lung, can provide a means to prevent the potentially metastatic cancer cell from becoming established in the lung. In general, however, the organ homing molecules of the invention are particularly useful for targeting a HPPS nanoparticle to a selected organ or tissue. Thus, the invention provides methods of treating a pathology in a selected organ or tissue by administering to a subject having the pathology a HPPS nanoparticle of the present invention.

Specific disorders of the lung, for example, can be treated by administering to a subject a HPPS nanoparticle comprising a lung homing molecule and a therapeutic agent. Since a lung homing molecule can localize to the capillaries and alveoli of the lung, disorders associated with these regions are especially amenable to treatment with a conjugate comprising the lung homing molecule. For example, bacterial pneumonia often originates in the alveoli and capillaries of the lung (Rubin and Farber, Pathology 2nd ed., (Lippincott Co.,1994)). Thus, a HPPS nanoparticle with a lung homing molecule and a suitable antibiotic can be administered to a subject to treat the pneumonia via a HPPS nanoparticle of the present invention. Similarly, cystic fibrosis causes pathological lesions in the lung due to a defect in the CFTR. Thus, administration of a HPPS nanoparticle with a lung homing molecule and a nucleic acid molecule encoding the CFTR provides a means for directing the nucleic acid molecule to the lung as an *in vivo* gene therapy treatment method.

The disclosed nanoscaffolds may be used for treating a pathology of the skin by administering to a subject having the pathology an HPPS nanoparticle comprising a skin homing molecule and a therapeutic agent. For example, a burn victim can be administered an HPPS nanoparticle comprising a skin homing molecule and an epithelial growth factor or platelet derived growth factor such that the growth factor is localized to the skin where it can accelerate regeneration or repair of the epithelium and underlying dermis. Furthermore, can be useful for treating skin pathologies caused by bacterial infections, particularly infections that spread through the hypodermis and dermis or that are localized in these regions, by administering to a subject a HPPS nanoparticle comprising a skin homing molecule and an antibiotic.

The disclosed nanoscaffolds may be used for treating a pathology of the pancreas by administering to a subject having the pathology an HPPS nanoparticle comprising a pancreas homing molecule and a therapeutic agent. In particular, since a pancreas homing molecule of the invention can localize to the exocrine pancreas, a pathology associated with the exocrine pancreas can be treated and, in some cases, may not adversely affect the endocrine pancreas. Nanoscaffolds can be particularly useful to treat acute pancreatitis, which is an inflammatory condition of the exocrine pancreas caused by secreted proteases damaging the organ. A HPPS nanoparticle comprising a pancreas homing molecule and a protease inhibitor can be used to inhibit the protease mediated destruction of the tissue, thus reducing the severity of the pathology. Appropriate protease inhibitors useful in such HPPS nanoparticles are those that inhibit enzymes associated with pancreatitis, including, for example, inhibitors of trypsin, chyrnotrypsin, elastase, carboxypeptidase and pancreatic lipase. Nanoscaffolds also can be used to treat a subject having a pancreatic cancer, for example, ductal adenocarcinoma, by administering to the subject a HPPS nanoparticle comprising a therapeutic agent linked to a molecule that homes to pancreas.

The disclosed nanoscaffolds may be used to treat a pathology of the eye, particularly the retina, by administering to a subject having the pathology an HPPS nanoparticle comprising a retina homing molecule and a therapeutic agent. For example, proliferative retinopathy is associated with neovascularization of the retina in response to retinal ischemia due, for example, to diabetes. Thus, administration of a conjugate comprising a retina homing molecule linked to a gene that stimulates apoptosis, for example, Bax, can be used to treat the proliferative retinopathy. Similarly, the disclosed nanoscaffolds may be used to diagnose or treat prostate, blood, ovary, breast, lymph node, adrenal gland, liver, or gut pathology using the appropriate organ or tissue homing molecules as disclosed herein.

The disclosed nanoscaffolds may be used for treating tissue infected with a variety of infective agents such as viruses, parasites, and/or bacteria (e.g. HIV, malaria... etc.). The homing molecule used with the HPPS nanoscaffold will typically include a ligand to a cell surface markers/receptors (e.g. proteins) expressed (or overexpressed) in the infected tissue.

The disclosed nanoscaffolds may be used for delivering a lipophilic compound, diagnostic agent or drug to a subject, target tissue, or organ comprising the steps of preparing a pharmaceutical formulation of the HPPS comprising a lipophilic drug in association with a lipid in an amount sufficient to form a complex with said lipophilic drug according to the methods of the invention and administering a therapeutically effective amount of the pharmaceutical formulation to said target tissue. The pharmaceutical formulation may be administered intravenously, intraarterially, intranasally such as by aerosol administration, nebulization, inhalation, or insufflation, intratracheally, intra-articularly, orally, transdermally, subcutaneously, rectally, or topically.

By "effective" or "therapeutically effective" amount is meant an amount that relieves (to some extent) one or more symptoms of the disease or condition in the patient. Additionally, by "therapeutically effective amount" is meant an amount that returns to normal, either partially or completely, physiological or biochemical parameters associated with or causative of a condition. Additionally, the effective amount may be one sufficient to achieve some other intended purpose, such as delivery of a radioimaging agent or other diagnostic agent to an organ or tissue.

Still further, the disclosed nanoscaffolds may be used for identifying a selected organ or tissue or diagnosing a pathology in a selected organ or tissue comprising the steps of preparing a pharmaceutical formulation of the HPPS comprising an appropriate targeting moiety and a diagnostic agent in association with a lipid in an amount sufficient to form a particle with said diagnostic agent according to the methods of the invention and administering to a subject a pharmaceutical formulation to said target organ or tissue.

"Diagnostic agent" refers to any agent which may be used in connection with methods for imaging an internal region of a patient and/or diagnosing the presence or absence of a disease in a patient. Exemplary diagnostic agents include, for example, radioactive and fluorescent labels and contrast agents for use in connection with ultrasound imaging, magnetic resonance imaging or computed tomography imaging of a patient. Diagnostic agents may also include any other agents useful in facilitating diagnosis of a disease or other condition in a patient, whether or not imaging methodology is employed.

The disclosed nanoscaffolds may be used for treating a subject suffering from a disorder selected from the group consisting of skin cancer, psoriasis, acne, eczema, rosacea, actinic keratosis, seborrheic dermatitis, and congenital keratinization disorders, in which any composition according to the methods of the invention is administered to the subject in need of such treatment by means of topical application.

The HPPS nanoparticles of the present invention can, as noted above, be used for topical application. Thus the disclosed nanoscaffolds may be used for treating one or more conditions of the skin selected from the group consisting of dry skin, photodamaged skin, age spots, aged skin, increasing stratum corneum flexibility, wrinkles, fine lines, actinic blemishes, skin dyschromias, and ichthyosis, comprising applying to the skin having said one or more condition any composition according to the invention, where the compound to be delivered is a known compound for treating such conditions, and is delivered in its known amount for treating such conditions. The term "topical application", as used herein, means to apply or spread the compositions of the present invention onto the surface of the skin.

The compound to be delivered in topical compositions of the present invention may comprise skin active ingredients. Non-limiting examples of such skin active ingredients include vitamin B3 compounds such as those described in PCT application WO 97/39733, published Oct. 30, 1997, to Oblong et al, flavonoid compounds; hydroxy acids such as salicylic acid; exfoliation or desquamatory agents such as zwitterionic surfactants; sunscreens such as 2-ethylhexyl-p-methoxycmnamate, 4,4'-t-butyl methoxydibenzoyl-methane, octocrylene, phenyl benzimidazole sulfonic acid; sun-blocks such as zinc oxide and titanium dioxide; anti-inflammatory agents; anti-oxidants/radical scavengers such as tocopherol and esters thereof; metal chelators, especially iron chelators; retinoids such as retinol, retinyl palmitate, retinyl acetate, retinyl propionate, and retinal; N-acetyl-L- cysteine and derivatives thereof; hydroxy acids such as glycolic acid; keto acids such as pyruvic acid; benzofuran derivatives; depilatory agents (e.g., sulfhydryl compounds); skin lightening agents (e.g., arbutin, kojic acid, hydroquinone, ascorbic acid and derivatives such as ascorbyl phosphate salts, placental extract, and the like); anti- cellulite agents (e.g., caffeine, theophylline); moisturizing agents; anti-microbial agents; anti-androgens; and skin protectants. Mixtures of any of the above mentioned skin actives may also be used. A more detailed description of these active ingredients is found in U.S. Pat. No. 5,605,894 to Blank et al. Preferred skin active ingredients include hydroxy acids such as salicylic acid, sunscreen, antioxidants and mixtures thereof. Topical applications can be practiced by applying a composition of the invention in the form of a skin lotion, cream, gel, emulsion, spray, conditioner, cosmetic, lipstick, foundation, nail polish, or the like which is intended to be left on the skin for some esthetic, prophylactic, therapeutic or other benefit.

### Methods of Making the HPPS nanoparticles of the Invention

### Nanoplatform Core Loading

The present disclosure provides methods of making the HPPS nanoparticles of the present invention. Prior art methods for incorporating agents into LDL and HDL particles are outlined in PCT Application Publication No. WO 2006/073419 to Zheng et al. (publication date: July 13, 2006; PCT Application No. PCT/US2005/011289).

In a preferred embodiment, HPPS nanoparticles are prepared by combining appropriate phospholipids with unsaturated cholesterol-esters in an organic solvent such as, for example, toluene, benzene, dichloromethane, and preferably chloroform, followed by evaporation of the solvent and vacuum-drying. Addition of buffer followed by sonication at around 40-60 °C produces an emulsion. Addition of appropriate scaffold peptides as noted above to the emulsion results in the preferred formation of spherical HPPS nanoparticles. If core-loading of HPPS nanoparticles is desired, the agents to be loaded into the core are initially combined with the phospholipids and cholesterol oleate, and the same procedure is followed.

### Attachment of Cell Surface Receptor Ligand

Following loading of the core of the HPPS nanoparticle with an active agent, the surface of the HPPS nanoparticle may be modified to attach a cell surface receptor ligand. Alternatively, the cell surface receptor ligand can also be incorporated concurrently with loading the active agent, hi some embodiments, the cell surface receptor ligand is covalently bonded to the scaffold peptides of the HPPS nanoparticles of the present invention.

Attachment of cell surface receptor ligands to the HPPS nanoparticles of the present invention can occur via standard techniques. The scaffold peptides of the HPPS nanoparticles may contain conjugable amino acid residues such as lysine, cysteine, serine, threonine, etc. In one embodiment, lysine residues are present and are coupled to the cell surface receptor ligand using known chemistry. For example, the ligand folic acid can be attached to an HPPS nanoparticle having lysine-containing scaffold peptides via increasing the pH by dialyzing HPPS nanoparticles against a buffer, i.e., NaH2PO4ZH3BO3 buffer. Folic acid-N-hydroxysuccinimide ester is then reacted with the HPPS nanoparticle at room temperature for 10 h. Upon completion of the reaction, the mixture is centrifuged to remove any degraded HPPS nanoparticles. In the final step, crude HPPS-FA can be dialyzed against EDTA buffer to adjust the pH to 7.4.

Remarkably, conjugation of cell surface receptor ligands such as folate and EGFR specific peptide to the scaffold peptides of the HPPS nanoparticles does not affect their ability to associate with phospholipids and form the nanocarrier. As well, preferably, conjugation of the folate and EGFR specific peptide to the lysine residues of the scaffold peptides places the homing molecule at or near the transition between a hydrophilic face and a hydrophobic face of the amphipathic α-helix.

In another embodiment, in addition to covalent attachment of cell surface receptor ligands to the scaffold peptides of the HPPS nanoparticles, cell surface receptor ligands may also be displayed on the surface of the HPPS nanoparticles via anchoring these cell surface receptor ligands to lipids which are incorporated into the phospholipids monolayer of the HPPS nanoparticles. In one embodiment, the lipid anchor may be 1,2-distearoyl-sn-glycero-3-phosphoethanolamine-N-[folate(polyethylene glycol)-2000] (DSPE-PEG (2000); Avanti).

The HPPS nanoparticles are taken up by cells through the SR-B1 receptor-mediated pathway. HDL targets SR-B1 (scavenger receptor type 1, also known as HDL receptor), and without being bound by theory it is thought that HPPS mimics HDL's SR-B1 specificity. As certain types of cancer (e.g., breast cancer) significantly overexpress SR-B1 receptors, the HPPS has the potential to selectively target malignant cells. Furthermore, the HPPS nanoparticles may be used to image vulnerable plaque, in the same manner as HDL-based Gd-MRI probes (Fayad group, Mt. Sinai New York: Frias et al. J. Am. Chem. Soc. 2004; 126:16316-7; Frias et al. Nano Lett. 2006; 6:220-4).

The Examples below illustrate the uptake of HPPS nanoparticles by cells both in vitro and in vivo through the SR-BI-mediated pathway. The initial evidence of cargo transport without HPPS internalization suggests that the HPPS nanocarrier is particularly useful for the direct cytosolic delivery of cancer diagnostics and therapeutics.

The examples below explain the invention in more detail. The following preparations and examples are given to enable those skilled in the art to more clearly understand and to practice the present invention. The present invention, however, is not limited in scope by the exemplified embodiments, which are intended as illustrations of single aspects of the invention only.

### EXAMPLES

### Example 1

### Preparation of Starting Materials

### 1) Size control peptide (scPep)

A few short peptide analogs of amphipathic helices, such as Ac-DWLKAFYDKVAEKLKEAF ("2F"), Ac-DWFKAFYDKVAEKFKEAF ("4F", also referenced herein as "+4F")), and Ac-FAEKFKEAVKDYFAKFWD (-4F) were synthesized on peptide synthesizer PS-3 (Protein Technologies, Inc.) by using Fmoc solid phase peptide synthesis (SPPS) protocol (Novabiochem, Resource for peptide synthesis: http://www.emdbiosciences.com/g.asp?f=NBC/peptideres.htm) using commercially available *N*-α-Fmoc protected amino acids, Sieber amide resin as a solid support and HBTU/HOBt as a carboxyl-group activating agent. After synthesis of the protected sequence, the N terminal Fmoc of peptide-resin was removed with 20% piperidine in *N,N*-dimethylformamide (DMF) to expose the terminal amine. The NH₂-peptide-resin was then capped with 10% pyridine in tetrahydrofuran with 10% acetic anhydride. The Ac-peptide-resin was further treated by 95% trifluoroacetic acid and 5% triisopropylsilane to remove the protected group on peptide sequence and cleave the solid support. After removing the cleaved solid resin by filtration, the filtrate was concentrated and precipitated by adding anhydrous ether to give scPep. The fluorescence of Tryptophan (W) was used to determine the scPep content of HPPS. Other methods known to those of skill in the art, such as amino acid analysis, may be used to determine the peptide content of HPPS. Lysine (K) having a free side-chain amine group was used for conjugating the targeting ligands.

### 2) EGFR-specific peptide (EGFp) as a targeting ligand for EGFR

A peptide sequence, Fmoc-YHWYGYTPQNVI, was synthesized. After final Fmoc removal, the peptide with N-terminal NH₂ group NH2-YHWYGYTPQNVI was cleaved from solid support. Three equalmolar amounts of the suberic acid bis-(N-hydroxysuccinimide ester) (Sigma Aldrich) was then conjugated to the N terminal of the EGFp in DMSO to form EGFp-NHS.

### 3) EGFp conjugated to phospholipid (EGFp-lipid).

First EGFp was reacted with suberic acid bis(N-hydroxysuccinimide ester) at a molar ratio of 1:5 in anhydrous dimethylsulfoxide (DMSO) in the presence of DIPEA to produce EGFp-N-hydroxysuccinimide ester (EGFp-NHS). The product EGFp-NHS was then incubated with 1,2-distearoyl-sn-glycero-3-phosphoethanolamine-N-[methoxy(polyethylene glycol)-2000] amine (DSPE-PEG (2000) amine, from Avanti) in DMSO at a molar ratio of 1:2. After a six hour incubation with mild mixing at room temperature, the sample was washed with an excess of diethyl ether. The precipitate was washed three more times with diethylether to remove any unreacted DSPE-PEG (2000) amine. The precipitate containing the final product was dried and resuspended in methanol.

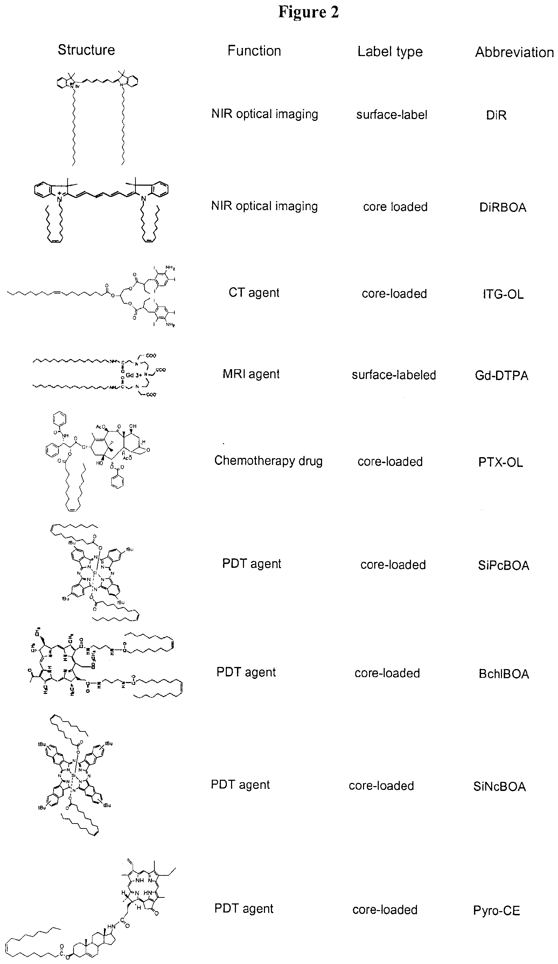

4) Compounds suitable for incorporation into the HPPS nanoparticle for diagnostic and/or therapeutic applications are set forth in Figure 2 and include near-infrared fluorescence (NIRF) imaging probes DiR-BOA (1,1'-dioctadecyl-3,3,3',3'-tetramethylindotricarbocyanine iodide bis-oleate) and DIR (1,1'-dioctadecyl-3,3,3',3'-tetramethylindotricarbocyanine iodide). DiR-BOA and DIR may be synthesized as a core-load and surface-loaded NIRF probe for HPPS, respectively. These two dyes have similar absorption and emission wavelength excitation (ex. 748nm, em. 782nm).

### 5) phospholipid

Phospholipids suitable for the HPPS preparation include but are not limited to DMPC (1,2-Dimyristoyl-sn-Glycero-3-Phosphocholine, POPC (1-palmitoyl-1-oleoyl-phosphatidylcholine) and EYPC (egg yolk phosphatidylcholine). All phospholipids were purchased from Avanti Polar Lipids Inc. (Alabaster, Alabama, USA).

### Example 2

### Method for HPPS Nanoparticle Preparation

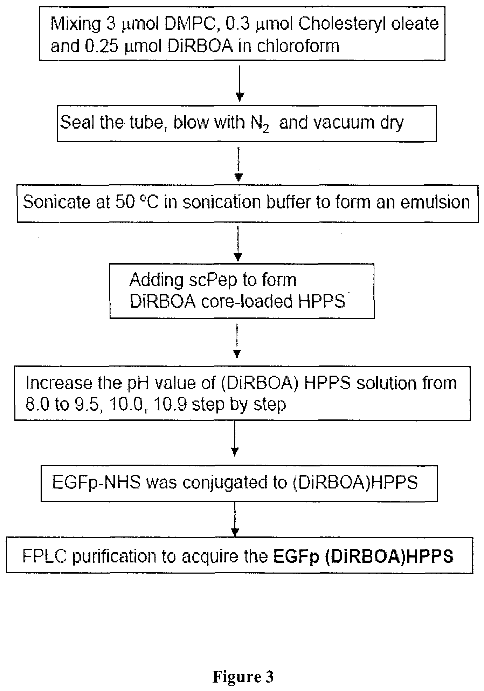

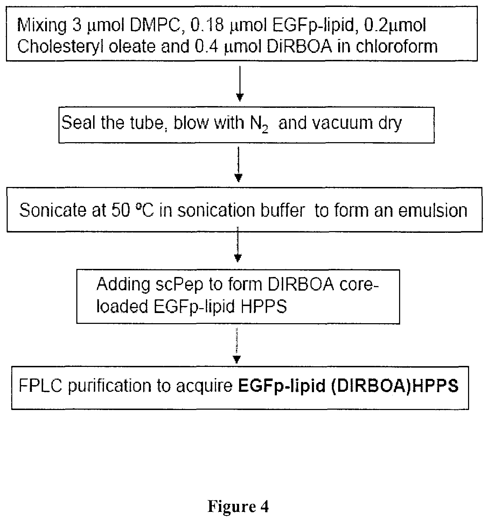

The HPPS is a macromolecule complex that is fully soluble in aqueous buffers, such as tris-saline (10mM tris-HCl, 150 mM NaCl, 1mM EDTA, pH 7.5). Tris-saline buffer was used as the solvent for HPPS in all of the experiments outlined below.
***1) HPPS:*** 3 µmol of DMPC and 0.3 µmol of cholesteryl oleate were dissolved in 0.5 mL of chloroform in a test tube. The solvent was slowly evaporated with N₂ and further dried by high vacuum. 1mL of buffer (10 mM Tris-HCl, pH 8.0, containing 0.1 M KCl, 1 mM EDTA) was added to the dry test tube afterwards and sonicated for 1 hr at 50°C to form emulsion. The scPep 0.8 µmol was added to solution to form HPPS particle. The particle was then purified by fast protein liquid chromatography (FPLC). The FPLC method will be described below.
***2) DiR-BOA core-loaded HPPS ((DiR-BOA) HPPS):*** Three µmol of DMPC and 0.3 µmol of cholesteryl oleate and 0.25 µmol of DiR-BOA were dissolved in 0.5 mL **of** chloroform in a test tube. The solvent was removed by slowly evaporated with N₂ and further dried by high vacuum. One mL of buffer (10 mM Tris-HCl, pH 8.0, containing 0.1 M KCl, 1 mM EDTA) was then added to the dried test tube and sonicated at 50°C for 1 hr to form emulsion. The scPep 0.8 µmol was added to the solution to assemble (DiR-BOA)HPPS particles. The particles were then purified by FPLC.
***3) DiR surface-labeled HPPS (DiR-HPPS):*** HPPS was labeled with a lipophilic near infrared dye DiR (1,1'-dioctadecyl-3,3,3',3'-tetramethylindotricarbocyanine iodide) (ex. 748nm, em. 782nm) (Molecular Probes, Inc., Eugene, OR) using a modified procedure based on the original method described by Pitas et al [J. Cell Biol. 1985, 100, 103-117]. Briefly, a stock solution of DiR was prepared by dissolving 3 mg (3.2 *µ*mol) of DiR in 1 mL dimethyl sulfoxide (DMSO); 0.1 mL of the stock was added to the 0.5 mL of HPPS solution (30 µM) to yield a final molar ratio of 40 of DiR to 1 of particle. After incubating this mixture in the dark for 18 hours at 37°C, the DiR surface-labeled HPPS (DiR-HPPS) was isolated by ultracentrifugation (49,000 rpm, 20 hours, 4°C, Beckman 50 Ti rotor), dialyzed against saline containing 0.01% EDTA, and filter-sterilized (0.45 µm, Water Millex HV units). The DiR loading in the DiR-HPPS was determined by checking the HPPS concentration (peptide concentration based on Tryptophan fluorescence at 320 nm) and DiR concentration (based on the DiR fluorescence at 782nm).
***4) EGFp conjugated scPep as targeting ligand for (DiR-BOA)HPPS, EGFp-(DiR-BOA)-HPPS (Type 1 EGFR-targeted HPPS):*** EGFp-conjugated DiR-BOA core-loaded HPPS ((DiR-BOA) HPPS) was synthesized according to the protocol depicted in Figure 3. Briefly, 3 µmol of DMPC, 0.2 µmol of cholesteryl oleate and 0.40 µmol of DiR-BOA were dissolved in chloroform in a test tube. The solvent was removed by slowly evaporated with N₂ and further dried by high vacuum. 1mL of buffer was added to the dried test tube afterwards and sonicated at 50°C for 1 hr to form an emulsion. The scPep was then added to the solution to assemble (DiR-BOA)HPPS particle. The pH of this particle solution was increased from 8.0 to 10.9 step by step by dialyzing against pH= 9.0, 10.0 10.9 of 0.1M NaH₂PO₄, 0.1M H₃BO₃ buffer respectively at 4°C. The active ligand molecules (1.8 µmol), such as EGFp-NHS or FA-NHS in anhydrous DMSO, was then added to the particles solution and the reaction mixture was on a shaker at room temperature. After 6 hours reaction, the mixture was then centrifuged at 500rpm at 4°C to remove any precipitate and subsequently dialyzed against EDTA buffer (0.3mM EDTA, 0.9% NaCl, pH 7.4) at 4°C overnight. Over the course of the dialysis, the pH of the EGFp-(DiR-BOA)-HPPS was returned to 7.4, and unreacted ligands starting materials were eliminated. This dialysis procedure was repeated until no spectrophotometrically detectable free ligand was present. The particle was then purified by FPLC (the FPLC method will be described below).
***5) EGFp-lipid as targeting ligand for (DiR-BOA)HPPS, EGFp-lipid (DiR-BOA) HPPS (Type 2 EGFR-targeted HPPS):*** As shown in Figure 4, the procedure to prepare EGFp-lipid (DiR-BOA) HPPS is similar to that of the (DiR-BOA) HPPS. The only difference being the addition of 0.18 µmol of EGFp-lipid (EGF peptide conjugated phospholipid) to 3 µmol of DMPC in the nanoparticles formulation.
***6) Folate conjugated scPep as targeting ligand for (DiR-BOA)HPPS, FA-(DiR-BOA)-HPPS (Type 1 FR-targeted HPPS):*** FA-(DiR-BOA)-HPPS was synthesized following the protocol described in Figure 3 (replacing EGFp with folate-NHS).
***7) Folate-lipid as targeting ligand for (DiR-BOA)HPPS, FA-lipid (DiR-BOA) HPPS (Type 2 FR-targeted HPPS):*** FA-lipid (DiR-BOA)-HPPS was synthesized following the protocol described in Figure 4 (replacing EGFp-lipid with folate-lipid).
**8) Full-length EGF as targeting ligand for (DiR-BOA) HPPS, *(-4F)EGF(DiR-BOA)HPPS:*** The full length recombinant human EGF was purchased from R&D Systems, Inc. (Minneapolis, Minnesota, USA). HPPS was formulated, as described above, with 30% of the phospholipid content as 1,2-distearoyl-sn-glycero-3-phosphoethanolamine-N-[methoxy(polyethylene glycol)-2000] maleimide (DSPE-PEG(2000) maleimide). EGF was incubated with Traut's reagent (2-Iminothiolane-hydrochloride)(Sigma) at pH 9.0 to introduce reactive sulfhydryl groups to EGF. The sulfhydryl-EGF (16 nmol) was then reacted with HPPS(DSPE-PEG(2000) maleimide)(0.1 µmol) for 24 hours at room temperature in a reaction volume of 1mL. Following the incubation period EGF-HPPS was purified by FPLC.

### Example 3

### Characterization of HPPS nanoparticles prepared using 4F

***1. Particle and payload stability:*** Particle and payload stability are essential for all nanoparticle-based drug delivery systems. Conventional lipid-based nanocarriers (e.g., liposomes and lipid emulsions) cannot maintain stability at ultra small size (<25nm). The HPPS nanocarrier system of the present invention displays remarkable stability. As shown in Table 4, over one month period, HPPS maintains their size and DiR-BOA payload, as determined by dynamic light scattering (see below) and fluorospectrophotometry. No payload leakage was observed after storage at 4 °C. Note the percentages in Table below indicate the proportion of the population of nanoparticles that have the indicated size.

**Table 4. Particle stability**

| HPPS Prep. on July 3, 2007 | Size Measurement July 9 | Size Measurement July 14 | Size Measurement Aug 4 | Fluorescence_{DIRBOA} July 19 | Fluorescence_{DIRBOA} Aug 4 |
|---|---|---|---|---|---|
| | 12.4nm, 99% | 10.9nm, 99% | 13.7nm, 99% | Em=1.07x10⁷ (at 777nm) | Em=1.14x10⁷ (at 777nm) |

| | | | | | |
|---|---|---|---|---|---|
| Note: The table displays the mean size, percent composition and DiR-BOA fluorescence intensity | | | | | |

When EGF peptide, full length EGF, and FA were used as targeting ligands, the stability of all three formulations appeared to be comparable over a one month period.

***2. Particle characterization:*** Four methods have been used to characterize the HPPS nanocarriers: 1) fast protein liquid chromatography (FPLC), 2) dynamic light scattering (DLS) (nanosizer), 3) transmission electron microscopy (TEM) and 4) circular dichroism (CD).

### Methods

*Electron Microscopy Studies.* A modern Hitachi H-7000 transmission electron microscope equipped with a digital image acquisition system was used to determine the morphology and the size of an aqueous dispersion of HPPS nanoparticles. Five microlitres of HPPS nanoparticle suspension was placed on carbon-coated 200 mesh copper grids and allowed to stand for 5 min. Excess sample was removed with lens paper, and 5 µL of a 2% saturated aqueous uranyl acetate was applied and allowed to stand for 20 sec. The stain was then drained off with filter paper, and the grid was air dried before digital images were taken. All electron microscopy supplies were purchased from Electron Microscopy Sciences (Fort Washington, PA).

*Dynamic Light Scattering.* The particle size distributions of HPPS particles were measured by light-scattering photon correlation spectroscopy (Zetasizer Nano-ZS90; Malvern Instruments, Malvern, UK) utilizing a 4.0 mW He-Ne laser operating at 633 nm and a detector angle of 90°. The data were modeled assuming spherical particles undergoing Brownian motion.

*Fast Protein Liquid Chromatography.* Following particle formation HPPS particles of the desired size was purified by gel filtration chromatrography using a Superdex 200 column (60 x 16 cm) with the Akta fast protein liquid chromatography (FPLC) system (Amersham Biosciences, Pittsburgh, PA). The particles were eluted with Tris-buffered saline (10 mM Tris-HCl, pH 7.5, containing 0.15 M NaCl, 1 mM EDTA) at a flow rate of 1mL/min. The sizes of the eluted particles were determined by comparing their retention time to those of proteins with known diameters: thyroglobulin (17.0 nm), apoferritin (12.2 nm), catalase (10.4 nm), bovine serum albumin (7.1 nm), alpha-chymotrypsin (4.2 nm), and ribonuclease A (3.8 nm).

Circular Dichroism (CD) Spectroscopy. Far-UV CD spectra of HPPS particles were recorded using a Jasco J-815 CD spectrometer. The CD spectra were recorded at 25 °C with a 1 nm step size from 260 to 190 nm. The data was collected over five consecutive scans and averaged. The alpha helix secondary structure of ScPep was monitored by changes in ellipticity at 222nm.

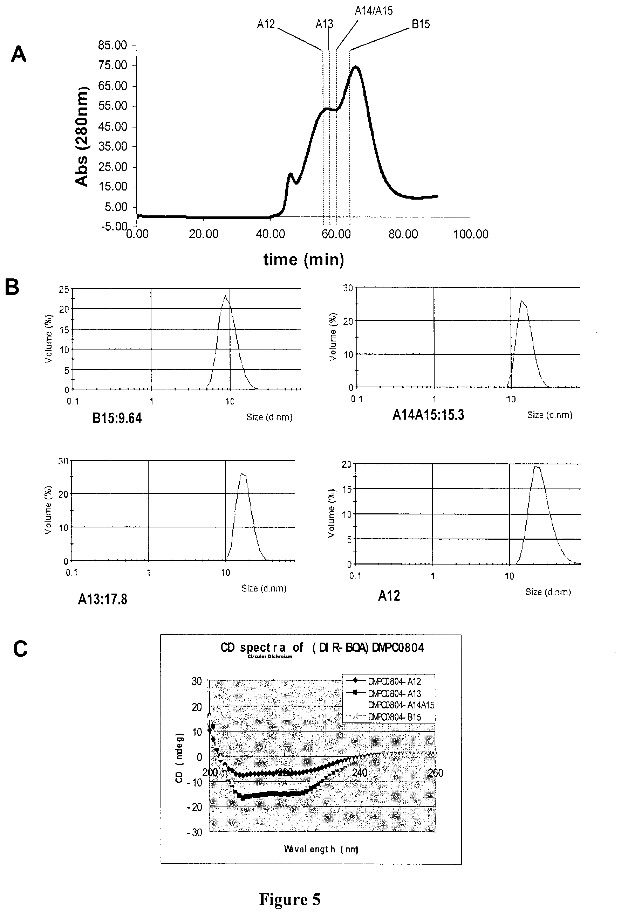

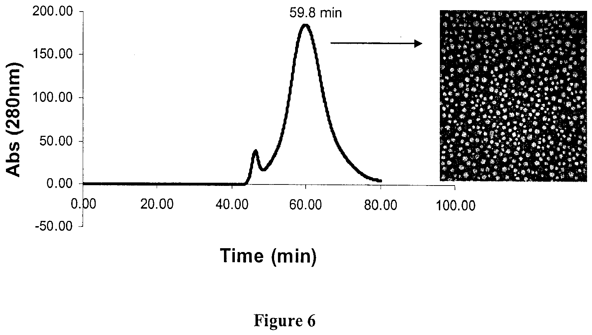

Figure 5 shows the FPLC, DLS and CD of untargeted DiR-BOA loaded HPPS prepared using 4F. From the FPLC (Figure 5A) different populations of HPPS particles can be collected as seen in Figure 5B. CD analysis of FPLC fractions reveals strong association of the scPep (presence of alpha-helix signature). A trend towards a higher content of alpha helix is seen for smaller particles. Figure 6 shows the FPLC measurement is consistent with TEM. The TEM displays the particles eluted at 60 min. FPLC shows a high yield of particles with a narrow distribution. The TEM confirm that the HPPS particles have a monodispersed spherical morphology. FPLC, TEM and DLS data (not shown) indicate that the EGFp conjugated HPPS particles range from 11-14 nm in diameter.

***3. Influence of DiR-BOA loading and targeting ligands on HPPS size:*** It was found that the size of HPPS can be controlled by their DiR-BOA payload. Here is an example: Two HPPS formulations were prepared as indicated below.

| PREPARATION #1 | PREPARATION # 2 |
|---|---|
| 3 µmol DMPC | 3 µmol DMPC |
| 0.2 µmol Cholesteryl oleate | 0.2 µmol Cholesteryl oleate |
| 0.2 µmol DIR-BOA | 0.4 µmol DIR-BOA |
| 0.8 µmol 4F | 0.8 µmol 4F |

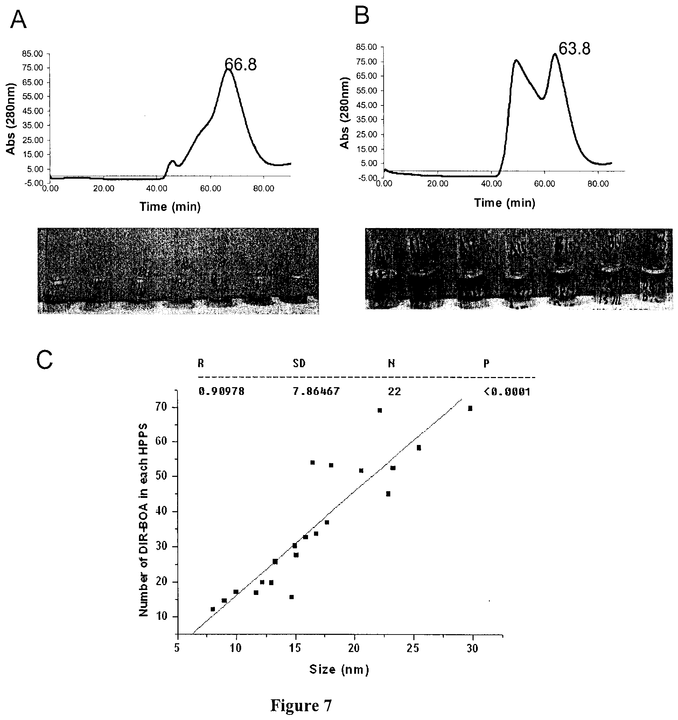

As shown in Figure 7, individual fractions of the FPLC profile were collected for each preparation. The formulation with the lower DIR-BOA loading (#1) produced more uniform distribution of smaller HPPS particles. Formulation #2 has a higher DiR-BOA payload (indicated by more intense blue coloration of collected fractions) and a more heterogeneous population of particles. The size of these HPPS particles were larger than that of formulation #1. These findings suggest that the size of the HPPS particle can be controlled/tuned by the cargo (DIR-BOA) loading.

Given that the size of the HPPS particle increases when one formulates the particle with higher doses of DiR-BOA, some control over the size of the HPPS 'end-product' can be achieved by adjusting the amount of core components (DiR-BOA & cholesterol-ester) added in the formulation. Thus an increasing/decreasing dose of core components will result in an increasing/decreasing size of HPPS particle. This is demonstrated in Figure 7C. A strong positive correlation coefficient is determined between the size of the HPPS and its DiRBOA payload (R=0.91).

When EGF peptide and full length EGF were used as targeting ligands for HPPS , the formed HPPS particle was slightly smaller (3-5 nm) than that prepared with FA. The peptide and full length EGF seemed to constrict the size of the HPPS.

### 4. Comparison of (DiR-BOA)HPPS formed with 4F and -4F:

The (DiR-BOA)HPPS nanoparticles were prepared as noted above, with 3.0 µmol DMPC, 0.2 µmol cholesteryl oleate, 0.2 µmol DiR-BOA, and 2 mg of either 4F or -4F.

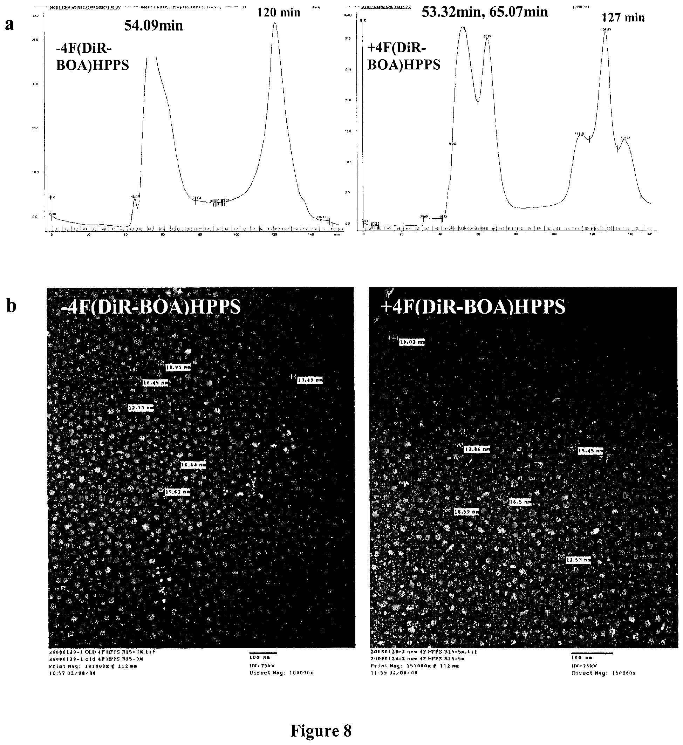

As shown in Figure 8 (a), the FPLC profile shows only one peak for -4F(DiR-BOA)HPPS, and two peaks for 4F(DiR-BOA)HPPS. Thus, both 4F and -4F can be used to form uniform nanoparticles.

As shown in Figure 8 (b), TEM imaging reveals that the spherical shape of (DiR-BOA)HPPS formed with -4F is more uniform than (DiR-BOA)HPPS formed with 4F.

### Example 4

### In vitro and in vivo evaluation of HPPS

Unless otherwise indicated, the scPep used in these studies was 4F.

Where concentrations of HPPS are mentioned below, these refer to the concentration of DiR-BOA carried by the HPPS. To determine the [DiR-BOA], a standard curve of DiR-BOA fluorescence versus DiR-BOA concentration was plotted. DiR-BOA was extracted from the HPPS particles with chloroform-methanol (2:1, v:v). The extraction was repeated twice and the chloroform layers pooled and dried with a speed vaccum. The residue was re-suspended in 1mL of chloroform-methanol (2:1, v:v) and the fluorescence was read on a fluorometer. From the fluorescence reading, the concentration of DiR-BOA in the HPPS particle could be determined from the standard curve.

The concentration of HPPS can also be determined based on peptide concentration. The peptide concentration of HPPS can be determined by the Bradford assay kit (Bio-Rad Laboratories, Inc. Hercules, CA). In such calculations, it was assumed that each HPPS particle contains 22 peptides.

For in vivo experiments, tris-saline was the buffer used to administer the HPPS. The volume of injection was typically 250-350 µL.

*Experimental Animals and Induction of Ectopic Tumors.* Various tumor xenograft models are described below. Here we present the general method used to prepare mice bearing tumor xenografts. Adult female nude mice (8-12 weeks old) were allowed free access to food and water throughout the study. Cancer cells (typically 3 x 10⁶ in PBS) were inoculated subcutaneously into the right flanks of nude mice. One to three weeks following tumor inoculation mice (with tumor size approximately 5mm) were used for designated studies.

### A. In vitro studies of EGFp(DiR-BOA)HPPS (Type 1 EGFR-targeted HPPS):

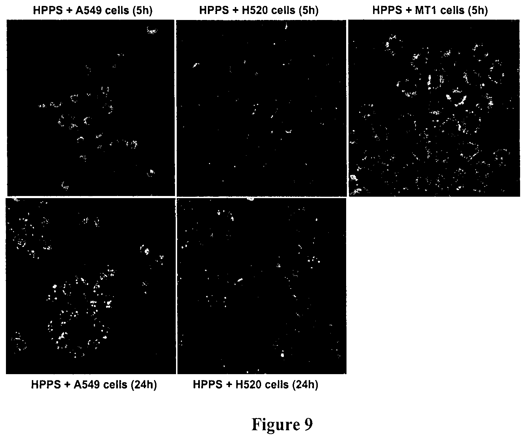

EGFR-targeting specificity of *EGFp(DiR-BOA)HPPS* was examined using confocal microscopy studies on cancer cells with different EGFR expression levels (A549 and
MT1: high expression, HepG₂: medium expression, H520: low expression). Confocal studies were performed using an Olympus FV1000 laser scanning confocal microscope.
A549, MT1 and H520 cells were incubated with 3µM of EGFp(DiR-BOA)HPPS (the concentration present here and that in the following *in vitro* and *in vivo* experiments are the DiR's concentration) in 0.2 mL of RPM1 1640 cell culture media with 10% fetal bovine serum (FBS). As shown in Figure 9, after a 5 and 24 hour incubation, A549 and
MT1 cells were shown to have higher EGFp(DiR-BOA) HPPS uptake than H520 cells, indicating EGFR-targeting specificity of HPPS.

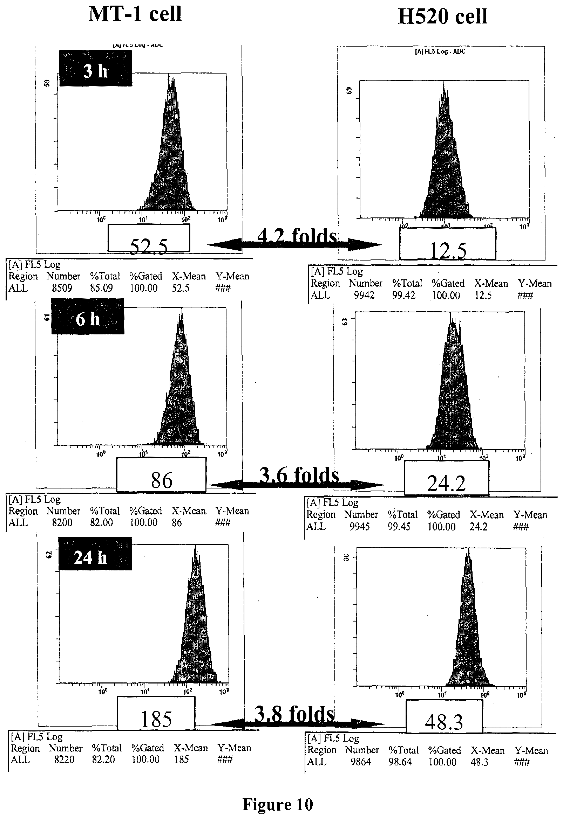

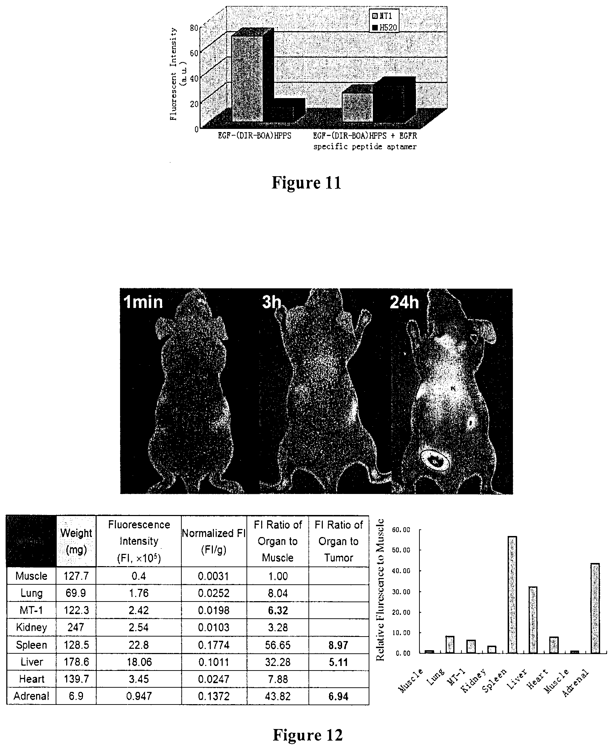

The EGFR-specificity of EGFp(DiR-BOA)HPPS was further confirmed using flow cytometry studies. In these studies, cells were incubated with EGFp(DiRBOA)HPPS for 3 to 24 hours and then analyzed on FV 500 flow cytometer. Figure 10 illustrates higher uptake of HPPS in MT-1 vs H520 cells after incubation with 1.1 µM of EGFp(DiR-BOA)HPPS. Figure 11 illustrates that using excess of EGFp(10 µM) as a competitive inhibitor, uptake of HPPS in MT-1 cells was reduced.

### B. In vivo studies of EGFp(DiR-BOA)HPPS

*In vivo NIR Fluorescence Imaging.* EGFp(DiR-BOA)HPPS was administered to mice via tail vein injection at a concentration of 5.2 nmol in 250 µL tris-saline. *In vivo* fluorescence imaging was performed before, 1, 5 and 24 hr post injection. Fluorescent images were obtained with a CRI Maestro™ *in vivo* imaging system. Mice were anesthetized with ketamine/acepromazine (50/5 mg/kg i.p.) and placed prone into the light-tight chamber of the Maestro imager. Fluorescent images were acquired with a deep red filter (ex=671-705nm, em=750nm long pass (730-950 in 10nm steps)) and an exposure time of 150 msec. A representative image is shown in Figure 12. By 24 hours the fluorescence intensity within the MT1 tumor is clearly visible indicating the preferential uptake of HPPS by the EGFR-expressing tumor.

For biodistribution studies, organs/tissue (MT-1 tumor, liver, spleen, heart, muscle, kidney and adrenals) of mice (24h post-injection of EGFp(DiR-BOA)HPPS) were harvested, weighed and homogenized in PBS. Homogenates were then combined with a 3-fold excess of a CHCl₃:MeOH (2:1) mixture, and vortexed for 2 min. Solutions were subsequently centrifuged at 3,000 rpm for 2 min. Using a Horiba JobinYvon Fluoromax-4 spectrofluorometer, the fluorescence intensities of the various samples were measured (ex. 748nm; em. 782nm). Fluorescence signals were normalized for sample weight and ratios are presented relative to muscle and tumor tissue (see Figure 12).

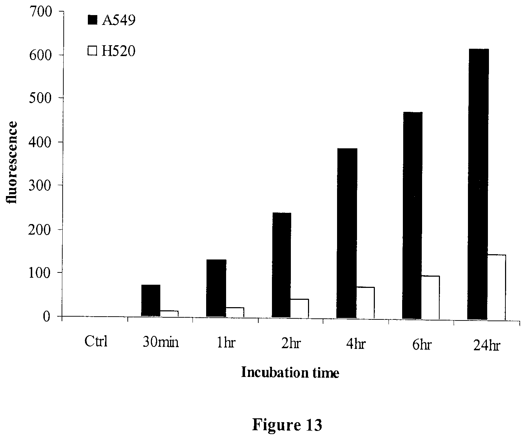

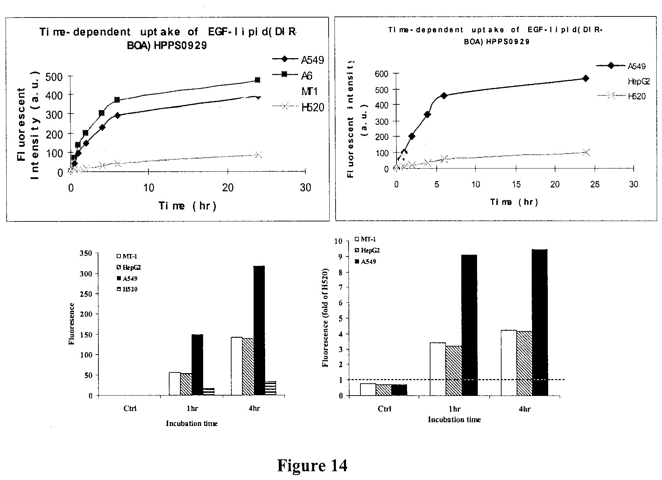

***C. In vitro studies of EGFp-lipid (DiR-BOA)HPPS (Type 2 EGFR-targeted HPPS):*** A series of flow cytometry experiments were performed with a Cytomics FC 500 Series Flow Cytometry System (Beckman Coulter, Inc. Mississauga, Ontario, Canada) to evaluate the EGFR-specificity of EGFp-lipid (DiR-BOA)HPPS. Cell culture experiments were carried out in 0.2 mL of RPM1 1640 cell culture media with 10% fetal bovine serum (FBS). First, as shown in Figure 13, A549 cells have much higher uptake of HPPS compared to H520 cells following a 3 hour incubation with 1.1 µM of EGFp-lipid (DiR-BOA)HPPS. These results suggest EGFR-specific accumulation of EGFp-lipid (DiR-BOA)HPPS in A549 cells. The EGFR-specificity was further demonstrated in Figure 14 showing time-dependent uptake of HPPS in cell lines with different EGFR expression levels.

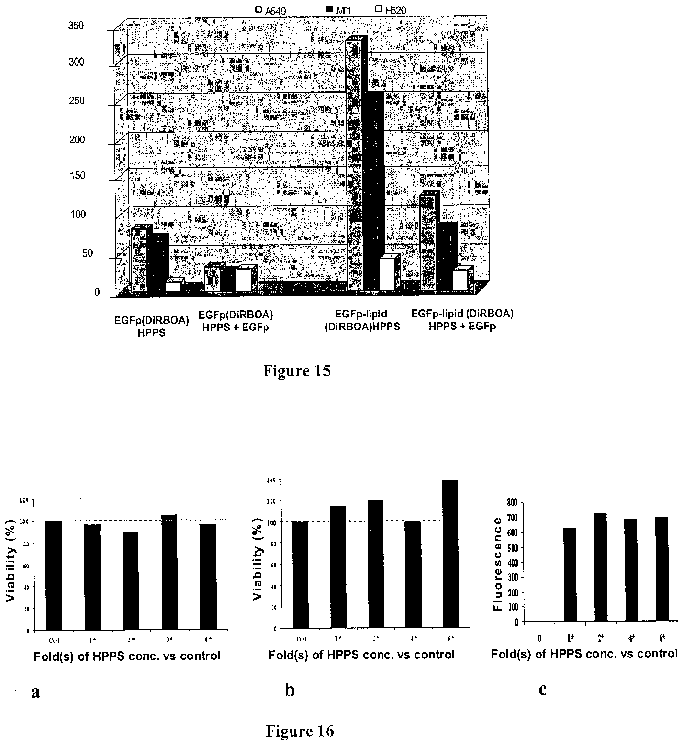

Further evidence of EGFR-specificity of EGFp-lipid(DiR-BOA)HPPS was demonstrated in Figure 15. The use of 9 fold excess of EGFp clearly inhibited the uptake of this Type 2 EGFR-targeted HPPS (right) in cells expressing high or medium level EGFR, which is consistent with the results obtained from the Type 1 EGFR-targeted HPPS (left).

***D. In vitro preliminary toxicity studies of EGFp-lipid (DiR-BOA)HPPS:*** To confirm that these biocompatible nanocarriers are indeed nontoxic, *in vitro* toxicity studies were performed using MTT and flow cytometry assays. To evaluate the toxicity, concentration-dependent HPPS uptake was studied in A549 cells by flow cytometry. Cell culture experiments were carried out in 0.2 mL of RPM1 1640 cell culture media with 10% fetal bovine serum (FBS). Since the standard drug dose of EGFp-lipid (DiR-BOA)HPPS used in all previous studies is 1.1 µM, the drug-dose dependent intracellular uptake of EGFp-lipid (DiR-BOA)HPPS was examined by increasing the drug dose. As shown in Figure 16c, 2-fold of the standard dose (2.2 µM) has resulted in uptake saturation. Therefore, it was decided to use up to 6-fold dose (6.6 µM) for toxicity evaluation. As can be seen in Figure 16a (MTT assay) and 15b (flow cytometry assay), no toxicity was observed in both MTT and flow cytometry experiments.

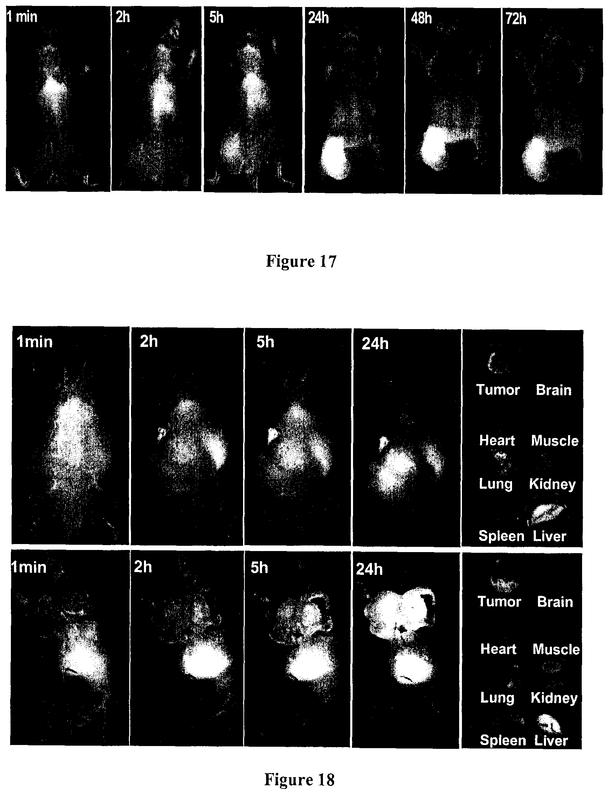

***E. In vivo studies of EGFp-lipid (DiR-BOA)HPPS:*** To evaluate the *in vivo* performance of the EGFp-lipid (DiR-BOA)HPPS, several tumor models were examined.
1) MT1 tumor xenografts: Nude female mice with MT1 tumors were examined by a CRI Maestro™ imager at several time points following the intravenous injection of 1.8 nmol (250 µl tris-saline) of EGFp-lipid (DiR-BOA)HPPS (Figure 17). For imaging methods see *in vivo* EGFp(DIR-BOA)HPPS study section 4B. It was observed that over the time course, the nanoparticles were shown to accumulate preferentially in MT1 tumor xenograft reaching maximum at 24-48h. At 72h, the HPPS began to clear from the tumor region.
2) Human lung primary tumor xenografts: The *in vivo* performance of EGFp-Lipid (DiR-BOA) HPPS (1.8 nmol)was also evaluated in primary tumor xenografts expressing EGFR. As shown in Figure 18, the top row showed a primary tumor on the kidney whereas the bottom row showed a primary tumor on the chest. Using *in vivo* real time NIR fluorescence imaging, it is clear that HPPS particles accumulate in both primary tumor sites over the 24 hours post injection period.. The results were also validated by imaging the excised tissues and tumors. Ex vivo tissue/tumor imaging was performed in a similar manner to whole animal imaging. (Fluorescent images were acquired on the Maestro imager with a deep red filter (ex=671-705nm, em=750nm long pass (730-950 in 10nm steps) and an exposure time of 150 msec).

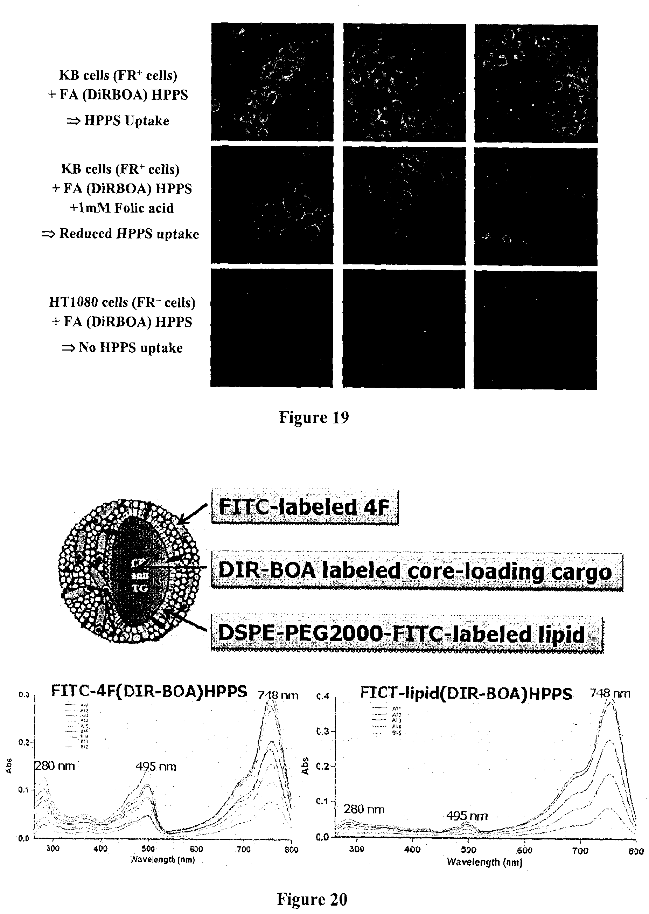

***F. In vitro studies of FA(DiR-BOA) HPPS:*** To validate the folate receptor (FR)-targeting of FA(DiR-BOA) HPPS, HPPS was incubated with cells which express the FR (KB cells, human epidermoid carcinoma cells) and cells which lack the FR (HT-1080 cells, human fibrosarcoma cells). Cell culture experiments were carried out in 0.2 mL of RPM1 1640 cell culture media with 10% fetal bovine serum (FBS). Confocal imaging studies (Figure 19) showed that, following a 4 h incubation with FA(DiR-BOA)HPPS (1100ng/mL), strong DiR-BOA fluorescence was seen throughout the KB cells cytoplasm indicating high uptake of the FR targeted HPPS nanoparticle (top row). FR-mediated uptake of FA(DiR-BOA) HPPS was confirmed by the additional control experiments. First, when KB cells were incubated with FA(DiR-BOA) HPPS along with excess FA, the uptake of FA(DiR-BOA) HPPS was competitively inhibited by FA (middle row). Secondly, the absence of significant fluorescence among HT-1080 cells (FR negative), suggests that avid uptake of FA(DiR-BOA) HPPS is dependent upon a FR mediated process (bottom row). Collectively these findings provide strong evidence that FA(DiR-BOA) HPPS was effectively targeted to FR.

***G. In vitro studies of (DiR-BOA) HPPS formed with -4F ("(-4F)(DiR-BOA)HPPS"):*** HDL targets and binds to SR-B1 (scavenger receptor type 1, also known as HDL receptor). It was thought that HPPS could mimic HDL's SR-B1 specificity, and that the core components of the HPPS could be taken up by cells via the SR-B1 receptor-mediated pathway.

(-4F)(DiR-BOA)HPPS nanoparticles were prepared as set forth in Example 3, part 4. Two cells lines were used to validate the hypothesis that the core material of HPPS (DiRBOA) is taken up by cells through the SR-B1 pathway: an SR-B1+ cell line known as ldlA(mSR-B1) and an SR-B1- cell line known as LDLA7 (Krieger, MIT). Carboxyfluorescein labelled phospholipid (DSPE-PEG-CF) (1,2-distearoyl-sn-glycero-3-phosphoethanolamine-N- [poly(ethylene glycol)2000-N'-carboxyfluorescein]) was used to trace the fate of the phospholipid component of HPPS following binding to SR-B1. Similarly fluorescein labelled 4F peptide was used to trace the fate of the peptide component (Figure 20).

DSPE-PEG-CF was purchased from Avanti Lipids. Fluorescein labeling of HPPS was carried out as suggested by the manufacturer's instructions. Briefly, HPPS was prepared as described above and dialyzed against 0.1M sodium carbonate buffer, pH 9. FITC (Sigma) was dissolved in anhydrous DMSO at a concentration of 1 mg/mL. FITC was then added to HPPS at a molar ratio of 1:1 (FITC:HPPS peptide). FITC was added very slowly with gentle and continuous stirring of the HPPS solution. The HPPS-FITC solution was incubated in the dark and the reaction continued for 8 hours at 4 °C. After this reaction period, the HPPS was dialyzed against Tris-buffered saline, and unbound FITC was removed by FPLC.

Meanwhile, near-infrared fluorescent probe DIR-BOA was core-loaded into HPPS with cholesteryl oleate to trace the fate of the cargo. The specific-uptake of HPPS cargo by SR-B1⁺ cells was quantified by flow cytometry using HDL-based competitive inhibition. The subcellular fate of each HPPS component was visualized by confocal imaging. Three dimensional images generated by confocal microscopy allow one to localize the fate of each labeled component, whether it is on the surface or interior (subcellular compartment) of the cell.

Figure 21 demonstrates the specificity of (DiR-BOA)HPPS for the SR-B1 receptor. Briefly, SR-B1 positive and negative cells (ldlA(mSR-B1)) and LDLA7, respectively), 20,000 of each, were cultured in Hams F-12 media (1% Penn/strep, 2mM L-glutamine and 5% FBS) for LDLA7 and Hams F-12 and + 300 µg/mL G418 for ldlA(mSR-B1). These cells were then incubated with (DiR-BOA)HPPS particles (1100 ng/mL) for 3 hours. In an additional inhibition experiment, cells were exposed to (DiR-BOA)HPPS and HDL (50 molar excess; 400 µg; 2.2 x10⁻⁴M). After the 3 hour incubation, confocal images show strong uptake of (DiR-BOA)HPPS in SR-B1 positive cells (ldlA(mSR-B1)) but not in SR-B1 negative cells (LDLA7). Furthermore, excess HDL effectively inhibits (DiR-BOA) HPPS uptake in ldlA(mSR-B1) cells.

Figure 22 illustrates the fate of each component of HPPS after binding to SR-B1. Similarly, 20,000 cells (ldlA(mSR-B1) were cultured in RPM1 1640 cell culture media with 10% fetal bovine serum. FITC-lipid or FITC-(-4F)(DiRBOA)HPPS (1100 ng/mL) was incubated with the cells for 1 hour.

Thereafter, confocal imaging was used to identify the subcellular localizations of each component of FITC-lipid(DiR-BOA)HPPS and FITC-(-4F)(DiR-BOA)HPPS after incubation with ldlA(mSR-B1) cells. It was found that HPPS can bind with SR-B1 and directly release DiR-BOA into the cytosol, whereas most of the labeled -4F peptide and phopholipid remained on the surface of the cells.

The release of DiR-BOA from the HPPS nanoparticle is further demonstrated in Figure 23. After a 3 hour incubation of ldlA(mSR-B1) with (DiR-BOA)HPPS, fluorescent signal from DiR-BOA can only be observed within the cytosol of unwashed cells. Little to no signal for DiR-BOA can be seen outside the cells due to the self-quenching effects DiR-BOA experiences in the small core of HPPS. Once (DiR-BOA)HPPS binds to SR-B1 and releases DiR-BOA in the target cell, the signal intensity of the DiR-BOA signal increases. The much larger intracellular volume of the cells allows the DIR-BOA molecules to disperse throughout the cytosol and thus overcome the self-quenching effect.

Flow cytometry studies were used to evaluate (-4F)(DiR-BOA)HPPS uptake in SR-B1⁺ and SR-B1⁻ cell lines. 5×10⁵ cells/well were cultured in 12-well plates for 2-3 days. RPMI 1640 medium with 10% FBS was used as the cell medium. Cells were treated for 5 hours under various conditions. FC500 was used to measure cellular fluorescence intensity.

To study the effect of -4F peptide on the uptake of particles, the HPPS made from DiR-BOA, DMPC and -4F was compared with an emulsion made from DiR-BOA and DMPC in SR-B1⁺ and SR-B1⁻ cell lines. The final concentration of DiR-BOA was 1,100 ng/mL.

Figure 24 demonstrates the requirement of -4F for efficient cellular uptake of (DiR-BOA)HPPS particles. A 1100 ng/mL solution of (DiR-BOA)HPPS and a peptide-free emulsion of DiR-BOA was exposed to ldlA(SR-B1) and LDLA7 cells for 3 hours. Cell culture experiments were carried out in Hams F-12 media (1% Penn/strep, 2mM L-glutamine and 5% FBS) for LDLA7 and Hams F-12 and + 300 µg/mL G418 for ldlA(mSR-B1). After the incubation period, cells were examined by flow cytometry. SR-B1 positive cells (ldlA(mSR-B1)) internalized much higher levels of (DiR-BOA)HPPS (3.5x) than the DiR-BOA emulsion. LDLA7 (SR-B1 negative cells) took up minimal amounts of either (DiR-BOA) HPPS or the emulsion.

Figure 25 displays the concentration-dependent uptake of -4F(DiR-BOA) HPPS in ldlA(mSR-B1) cells. Cell culture experiments were carried out in 0.2 mL of Hams F-12 and + 300 µg/mL G418 for ldlA(mSR-B1). SR-B1 positive cells (ldlA(mSR-B1) were incubated for 3 hours with varying concentrations of -4F(DiR-BOA) HPPS ranging from 13.8 to 1100 ng/mL. Flow cytometry analysis revealed that the uptake of -4F(DiR-BOA) HPPS was proportional to the incubation dose of the particle. Increasing amounts of -4F(DiR-BOA) HPPS resulted in increased amounts of DiR-BOA with the ldlA(mSR-B1) cells. Saturated uptake of -4F(DiR-BOA) HPPS in ldlA(mSR-B1) cells occurred at 440ng/mL.

HDL inhibits the uptake of (-4F)(DiR-BOA)HPPS in a dose dependent manner (Figure 26). Cell culture experiments were carried out in 0.2 mL of Hams F-12 and + 300 µg/mL G418 for ldlA(mSR-B1). ldlA(mSR-B1) cells were incubated for 3 hours with HDL and (-4F)(DiR-BOA)HPPS (molar ratios ranging from 0:1 to 50:1; HDL concentrations ranged from 9.0 x 10⁻⁷ to 2.2 x10⁻⁴ molar). Increasing concentrations of HDL inhibited (-4F)(DiR-BOA)HPPS uptake in ldlA(mSR-B1) cells with increasing effectiveness. Near to complete inhibition of (-4F)(DiR-BOA)HPPS cellular uptake was observed at a 50:1 molar excess of HDL. These results further confirm the SR-B1 mediated uptake of (-4F)(DiR-BOA)HPPS.

Figure 27 further illustrates the selective uptake of (-4F)(DiR-BOA)HPPS in SR-B1⁺ cells, as well as the inhibition of this uptake by HDL. 2×10⁴ cells/well SR-B1⁺ and SR-B1⁻ cells were cultured in an 8 well cover slides chamber for 2-3 days. RPMI 1640 medium with 10% FBS was used as cell medium. One day before the confocal study, the cell medium was changed to RPMI 1640 medium without FBS. Cells were treated for 5 hours under various conditions.

Scanning confocal microscopy was also used to evaluate the uptake of (-4F)(DiR-BOA)HPPS in cells (Figure 27). Cell culture experiments were carried out in Hams F-12 media (1% Penn/strep, 2mM L-glutamine and 5% FBS) for LDLA7 and Hams F-12 and + 300 µg/mL G418 for ldlA(mSR-B1). Briefly 2.39 µg/mL of (-4F)(DiR-BOA)HPPS was incubated with ldlA(mSR-B1) and LDLA7 cells for 3 hours. Following the incubation period, marked fluorescence signal was observed in ldlA(mSR-B1) but not in LDLA7. In a similar inhibition study, HDL (50 fold excess; 2.2 x10⁻⁴ M) was incubated along with (-4F)(DiR-BOA)HPPS. After the 3 hours of incubation, no fluorescence was detected in ldlA(mSR-B1) indicating that HDL completely blocked the uptake of (-4F)(DiR-BOA)HPPS in ldlA(mSR-B1). No fluorescence was detected in LDLA7 cells.

Figure 28 shows the efficacy of (4F and -4F)(DiR-BOA)HPPS uptake in ldlA(mSR-B1) cells. Cell culture experiments were carried out in 0.2 mL of Hams F-12 and + 300 µg/mL G418 for ldlA(mSR-B1). Fourteen to two hundred and twenty ng/mL of (4F and -4F)(DiR-BOA)HPPS were incubated with ldlA(mSR-B1) cells for 3 hours. Flow cytometry revealed that both formulations of HPPS were taken up in ldlA(mSR-B1) cells in a dose dependent manner. (-4F)(DiR-BOA)HPPS displayed slightly higher cellular uptake compared to its 4F counterpart.

***H. In vivo studies of (DiR-BOA) HPPS formed with -4F ("(-4F)(DiR-BOA)HPPS")**: In vivo* imaging using the Maestro system was performed on nude mice bearing human KB (SR-BI⁺) tumor xenograft (Figure 29). Optical images were collected up to 72 hours post injection of -4F(DiR-BOA)HPPS (5.2 nmol; 350 µL tris-saline). DiR-BOA fluorescent signal can be seen to selectively localize in the KB tumor xenograft as early as 8 hours post injection. Fluorescent images of the excised organs are also displayed.

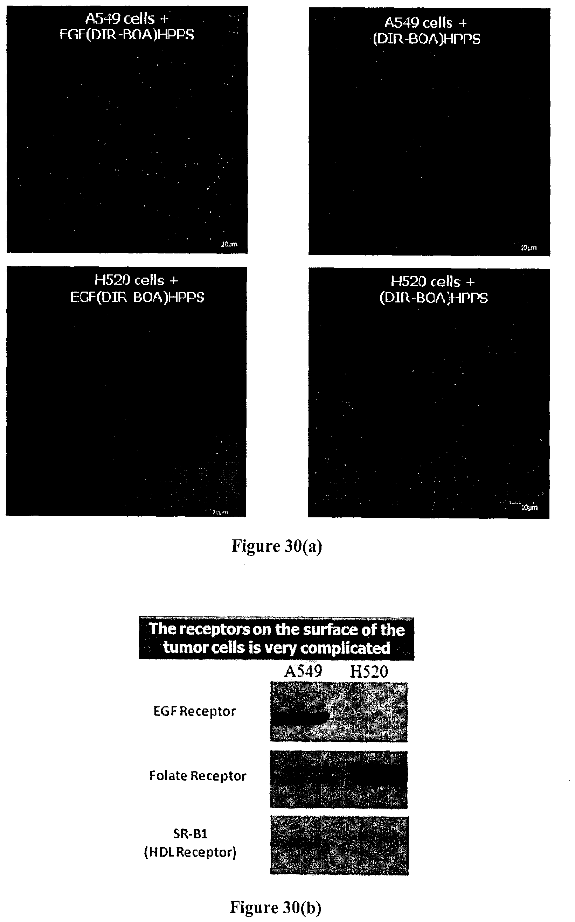

***I. In vitro studies of (-4F)EGF(DiR-BOA)HPPS (full-length EGF as targeting ligand):*** EGFR-targeting specificity of (-4F)EGF(DiR-BOA)HPPS was examined using confocal microscopy studies on cancer cells with different EGFR expression levels (A549: high expression, H520: low expression). Cell culture experiments were carried out in 0.2 mL of RPM1 1640 cell culture media with 10% fetal bovine serum (FBS). Briefly, confocal studies were performed on A549 and H520 cells incubated with 1.0 µM of (-4F)EGF(DiR-BOA)HPPS (the concentration present here and that in the following *in vitro* and *in vivo* experiments are the DiR's concentration). Figure 30(a) shows that A549 cells have higher (-4F)EGF(DiR-BOA)HPPS uptake than H520 cells. However, it should be noted that the uptake of (-4F)(DiR-BOA)HPPS is also notably higher in A549 than H520 cells. Western blot analysis of various surface receptor proteins for A549 and H520 were performed (Figure 30b). EGFR was over expressed in A549 cells relative to H520 cells. SR-B1 expression was comparable between the two cell lines, whereas folate receptor levels appears over-expressed in H520 compared to A549.

(-4F)(DiR-BOA)HPPS and (-4F)EGF(DiR-BOA)HPPS (1.0 µM) were incubated with H520, A549 and EGFR-GFP-A549 cells for 3 hours. Cell culture experiments were carried out, as previously described, in 0.2 mL of RPM1 1640 cell culture media with 10% fetal bovine serum (FBS). Flow cytometry data shows that (-4F)(DiR-BOA) HPPS has equally taken up in all 3 cells. HDL (400 µg) also equally inhibited the uptake of these particles in these cells (see Figure 31). The uptake of (-4F)EGF(DiR-BOA)HPPS in A549 and EGFR-GFP-A549 greatly surpassed that of H520. The presence of HDL reduced but did not eliminate the uptake of (-4F)EGF(DiR-BOA)HPPS in the three cell lines. The residual uptake of (-4F)EGF(DiR-BOA)HPPS in A549 and EGFR-GFP-A549 cells reflects the EGFR mediated uptake of this particle in EGFR/SR-B1 positive cells.

In order to determine what role SR-B1 may contribute to the uptake of EGF-HPPS, SR-B1 negative cells, LDLA7, were co-transfected with genes for EGFR and green fluorescent protein (GFP). This experiment removes any contribution SR-B1 may make to the cellular uptake of EGF-HPPS. Cell culture experiments were carried out, as previously described, in Hams F-12 media (1% Penn/strep, 2mM L-glutamine and 5% FBS). Following a 3 hour incubation with (-4F) EGF(DiR-BOA)HPPS (1.0 µM), EGFR-GFP-LDLA7 cells displayed a strong signal of DiR-BOA within the cytosol while the GFP signal for the EGFR was visualized on the outer plasma membrane (Figure 32). Confocal studies were performed to evaluate the effect of HDL on the uptake of (-4F)EGF(DiR-BOA)HPPS in EGFR-GFP-LDLA7 cells (Figure 33). As can be seen in the GFP channel, LDLA7 cells that were successfully transfected with EGFR and GFP constitutively express fluorescence. Following treatment with (-4F)EGF(DiR-BOA)HPPS (1.0 µM), DiRBOA signal can be detected within the cells (DiR channel). When an excess of HDL (400µg) is added during the treatment with (-4F)EGF(DiR-BOA)HPPS, fluorescence signal from DiRBOA can still be seen throughout the treated cell. The degree of (-4F)EGF(DiR-BOA)HPPS uptake in EGFR-GFP-LDLA7 cells is similar with and without HDL competition. Thus, HDL has no effect on the uptake of (-4F)EGF(DiR-BOA)HPPS in EGFR-GFP-LDLA7 cells. These findings demonstrate the specific EGFR mediated uptake of (-4F)EGF(DiR-BOA)HPPS in EGFR-GFP-LDLA7 cells.

EGFR-GFP-A549 and H520 cells were incubated with 1.0 µM (-4F)EGF(DiR-BOA)HPPS for 3 hours. Cell culture experiments were carried out in 0.2 mL of RPM1 1640 cell culture media with 10% fetal bovine serum (FBS). Following the 3 hour incubation period, confocal imaging showed strong fluorescence in EGFR-GFP-A549 cells (Figure 34). The presence of excess HDL slightly decreased the fluorescent signal in these cells. The combination of excess HDL and EGF markedly reduced the fluorescent signal in the EGFR-GFP-A549 cells to levels similar to that seen in the cell alone controls. None of the H520 treatment groups displayed DiR-BOA fluorescence. This indicates that H520 did not take up any (-4F)EGF(DiR-BOA)HPPS.

In Figure 35 (-4F)EGF(DiR-BOA)HPPS (1.0 µM) was incubated with A549 and H520 cells. Cell culture experiments were carried out in 0.2 mL of RPM1 1640 cell culture media with 10% fetal bovine serum (FBS). HDL (400µg) was also added during this incubation period to abolish any contributions SR-B1 may have on the uptake process. The cells were monitored 1, 3 and 6 hours post HPPS exposure. In the presence of HDL, A549 displayed much higher uptake of (-4F)EGF(DiR-BOA)HPPS than H520, however, in the absence of HDL(insert), the difference in the fluorescent signal between A549 and H520 was markedly diminished. These findings suggest that SR-B1 does contribute to the uptake of (-4F)EGF(DiR-BOA)HPPS in SR-B1/EGFR positive cells.

***J. In vivo studies of (-4F)EGF(DiR-BOA)HPPS (full-length EGF as targeting ligand):*** Figure 36 shows *in vivo* optical images of nude mice bearing A549 and H520 tumor xenografts at various times following (-4F)EGF(DiR-BOA)HPPS (5.2 nmol in tris-saline) intravenous injection. In the early post injection period, fluorescent signal is seen throughout the animal. By 22 hours, focal signal can be seen in the A549 and H520 while fluorescent signal in the rest of the body is greatly diminished. This strong fluorescent signal is retained in the A549 tumor throughout the study period; however, by day 3, the signal in the H520 tumor washes out. These results suggest that the EGFR in the A549 tumor helps to accumulate and retain (-4F)EGF(DiR-BOA)HPPS while the uptake in H520 was nonspecific and transient.

***K. In vivo studies of (-4F)EGF(DiR-BOA)HPPS in KB tumor xenografts:*** Figure 37A shows *in vivo* optical images of nude mice bearing KB tumor xenografts at various times following i.v. injection of (-4F)EGF(DiR-BOA)HPPS (5.2 nmol in tris-saline). Only tumor tissue was enhanced by DiR-BOA fluorescence that reaches a maximum at 48h. In Figure 37B the biodistribution of (-4F)(DiR-BOA)HPPS (HPPS) (5.2 nmol in tris-saline), (-4F)EGF(DiR-BOA)HPPS (EGF-HPPS) (5.2 nmol in tris-saline) and EGF-HPPS + HDL (5.2 nmol and 0.14µmol in tris-saline) particles were assessed in mice bearing KB xenografts. The biodistribution profiles all show high uptake of DiR-BOA in tumor over all other normal organs except liver. The tumor uptake of EGF-HPPS was greater than that of HPPS or EGF-HPPS+HDL. In Figure 37C the circulation half-life of HPPS, (-4F)PEG(DiR-BOA)HPPS (PEG-HPPS) (**PEG**-HPPS was synthesized following the protocol described in Figure 3 (replacing EGFp with 1,2-distearoyl-sn-glycero-3-phosphoethanolamine-N-[methoxy(polyethylene glycol)-2000] (DSPE-PEG(2000)) and EGF-HPPS was determined in normal nude mice. All animals received an intravenous injection of 5.2 nmol of the respective nanoparticle in tris-saline. Blood samples were collected before the nanoparticle injection and various times after the injection. DiR-BOA was extracted from the blood samples and its fluorescence was determined on a fluorimeter. The circulation half-life was determined to be approximately 14h, comparable with the best engineered nanocarrier to date. Finally Figure 37D shows mice bearing dual KB tumor xenografts that have been stably transfected with red fluorescent protein. Following an injection of EGF-HPPS (5.2 nmol in tris-saline) one can see that the fluorescence of DiR-BOA closely overlaps with the signal of red fluorescent protein. These results indicate that EGF-HPPS nanoparticles can effectively target tumors expressing EGFR.

### L. In vivo validation of specific EGFR mediated cellular uptake of (-4F)EGF(DiR-BOA)HPPS versus non-specific tissue accumulation

Nude mice bearing KB (EGFR +'ve) and H520 (EGFR -'ve) tumor xenografts were intravenously co-injected with native HDL (0.14 µmol in tris-saline) immediately followed with (-4F)EGF(DiR-BOA)HPPS (5.2 nmol in tris-saline). Forty-eight hours after the injection mice were sacrificed and the tumors were excised. Tissues were washed with phosphate buffered saline (PBS) and then 40 mg of each tissue was subsequently used for 'tissue analysis'. Similarly, 150 mg of each tissue was used for 'cell analysis'.

**Tissue analysis.** Tissue samples were homogenized in PBS followed by chloroform:methanol extraction as described earlier in section B (*"In vivo studies of EGFp(DiR-BOA)HPPS"*). Tissue fluorescence of the extract was measured and presented as fluorescence per unit (mg) tissue.

**Cell analysis.** Following homogenization, the samples were filtered with nylon mesh (pore size 70 µm) to remove debris. The filtered samples (liberated cells) were then washed twice with PBS and treated with red blood cell lysis buffer (0.1% potassium bicarbonate, 0.8% ammonium chloride, 0.1 mM EDTA) for 10 min to remove red blood cells. Cells were counted with a hemocytometer using an inverted microscope. Thereafter, the cells were extracted with chloroform:methanol (as described above). Fluorescence of the cell extract was measured and presented as fluorescence per million cells.

A comparison of (-4F)EGF(DiR-BOA)HPPS uptake at the tissue and cell level is presented in Figure 38. When viewed from the tissue level, KB and H520 tumor xenografts accumulate (-4F)EGF(DiR-BOA)HPPS to an equal degree. Conversely when examined at the cell level, KB cells take up (-4F)EGF(DiR-BOA)HPPS to a greater degree (2x) than H520.

The tissue accumulation of (-4F)EGF(DiR-BOA)HPPS reflects a composite of extracellular trapping of the particle due to enhanced permeability and retention mechanisms as well as intracellular uptake of the particle in epithelial cells. When one isolates the cells and removes contributions from extracellular factors, different findings emerge. A clearer picture of EGFR mediated uptake of HPPS is revealed. In addition, contributions of SRBI to HPPS uptake are also minimized due to the co-injection of native HDL. Collectively, these findings validate the EGFR targeting specificity of (-4F)EGF(DiR-BOA)HPPS nanoparticles.

## Claims

1. A non-naturally occurring peptide-lipid nanoscaffold capable of being taken up by cells through the SR-B1 receptor-mediated pathway comprising:
a) at least one phospholipid;
b) at least one unsaturated lipid ester, and
c) at least one peptide between 6 and 30 amino acids in length, the peptide comprising an amino acid sequence capable of forming at least one amphipathic α-helix;
wherein the components a), b) and c) associate to form the peptide-phospholipid nanoscaffold.

2. The peptide-lipid nanoscaffold of claim 1, further comprising:
d) at least one homing molecule; and
wherein the components a), b), c) and d) associate to form the peptide-phospholipid nanoscaffold.

3. The peptide-lipid nanoscaffold of claim 2, wherein the at least one homing molecule is covalently bound to the at least one phospholipid.

4. The peptide-lipid nanoscaffold of claim 2, wherein the at least one homing molecule is covalently bound to the at least one peptide.

5. The peptide-lipid nanoscaffold of claim 4, wherein the at least one homing molecule is bound to the peptide at an amino acid at or near the transition between a hydrophilic face and a hydrophobic face of the amphipathic α-helix.

6. The peptide-lipid nanoscaffold of claim 5, wherein the amino acid of the peptide bound to the homing molecule is selected from the group consisting of Lysine, Cysteine, Threonine and Serine, preferably Lysine.

7. The peptide-lipid nanoscaffold of claim 2, wherein the at least one homing molecule is at least one active cell surface receptor ligand.

8. The peptide-lipid nanoscaffold of claim 7, further comprising:
e) at least one active agent; and
wherein the active agent is released into the cytosol of said target cell after being taken up by cells through the to SR-B1 receptor-mediated pathway.

9. The peptide-lipid nanoscaffold of claim 8, wherein the peptide is an analog of an amphipathic α-helix.

10. The peptide-lipid nanoscaffold of claim 8, wherein the peptide is selected from the group consisting of 2F, 4F, the reverse sequence of 2F and the reverse sequence of 4F.

11. The peptide-lipid nanoscaffold of claim 8, wherein the at least one amphipathic α-helix or peptide is between 8 and 28 amino acids in length, between 10 and 24 amino acids in length, between 11 and 22 amino acids in length, between 14 and 21 amino acids in length, between 16 and 20 amino acids in length or 18 amino acids in length.

12. The peptide-lipid nanoscaffold of claim 8, wherein the phospholipid is selected from the group consisting of phosphatidylcholine (preferably DMPC (1,2-Dimyristoyl-sn-Glycero- 3-Phosphocholine), POPC (1-palmitoyl-1-oleoyl-phosphatidylcholine) and EYPC (egg yolk phosphatidylcholine)), lysophosphatidylcholine, phosphatidylethanolamine, phosphatidylserine, phosphatidylinositol and combinations of the foregoing.

13. The peptide-lipid nanoscaffold of claim 8, wherein the nanoscaffold is between 5-50nm in diameter, between 5-40nm in diameter, between 5-30nm in diameter, between 5-25nm in diameter, between 5-20nm in diameter or between 10-15nm in diameter.

14. The peptide-lipid nanoscaffold of claim 8, wherein the at least one active agent is bound to at least one lipid anchor, preferably selected from the group consisting of a cholesterol oleate moiety, cholesteryl laurate moiety, phytol moiety and preferably an oleate moiety and an unsaturated cholesterol-ester moiety; and preferably bound by an ester bond.

15. The peptide-lipid nanoscaffold of claim 8, wherein the homing molecule is selected from the group consisting of a peptide, an aptamer, an antibody and an antibody fragment.

16. The peptide-lipid nanoscaffold of claim 8, wherein the active agent is a lipophilic compound.

17. The peptide-lipid nanoscaffold of claim 8, wherein the active agent is a molecule comprising a lipophilic and a hydrophilic component.

18. The peptide-lipid nanoscaffold of claim 8, wherein the active agent is a diagnostic agent, imaging agent or a therapeutic agent.

19. The peptide-lipid nanoscaffold of claim 1, wherein the amino acid sequence comprises consecutive amino acids of an apoprotein in a forward or reverse direction, preferably selected from the group consisting of apoB-100, apoB-48, apoC, apoE and apoA.

20. The peptide-lipid nanoscaffold of claim 1, further comprising one or more triacylglycerols, sterols, sterol esters, or combinations thereof.

21. The peptide-lipid nanoscaffold of claim 1, wherein the unsaturated lipid ester is an unsaturated sterol ester.

22. The peptide-lipid nanoscaffold of claim 21, wherein the unsaturated sterol ester is an unsaturated cholesterol ester.

23. The peptide-lipid nanoscaffold of claim 22, wherein the unsaturated cholesterol ester is cholesteryl oleate.

## Patentansprüche

1. Nicht natürlich vorkommendes Peptid-Lipid-Nanogerüst, das von Zellen über den vom SR-B1-Rezeptor vermittelten Pfad aufgenommen werden kann, umfassend:
a) mindestens ein Phospholipid,
b) mindestens einen ungesättigten Lipidester und
c) mindestens ein Peptid mit einer Länge zwischen 6 und 30 Aminosäuren, wobei das Peptid eine Aminosäuresequenz umfasst, welche mindestens eine amphipathische α-Helix bilden kann,
wobei die Komponenten a), b) und c) sich unter Bildung des Peptid-Phospholipid-Nanogerüstes verbinden.

2. Peptid-Lipid-Nanogerüst nach Anspruch 1, ferner umfassend
d) mindestens ein Homing-Molekül,
wobei die Komponenten a), b), c) und d) sich unter Bildung des Peptid-Phosphorlipid-Nanogerüstes verbinden.

3. Peptid-Lipid-Nanogerüst nach Anspruch 2, wobei das mindestens eine Homing-Molekül an das mindestens eine Phospholipid kovalent gebunden ist.

4. Peptid-Lipid-Nanogerüst nach Anspruch 2, wobei das mindestens eine Homing-Molekül an das mindestens eine Peptid kovalent gebunden ist.

5. Peptid-Lipid-Nanogerüst nach Anspruch 4, wobei das mindestens eine Homing-Molekül an das Peptid an einer Aminosäure an oder nahe dem Übergang zwischen der hydrophilen Oberfläche und der hydrophoben Oberfläche der amphipathischen α-Helix gebunden ist.

6. Peptid-Lipid-Nanogerüst nach Anspruch 5, wobei die an das Homing-Molekül gebundene Aminosäure des Peptids aus der aus Lysin, Cystein, Threonin und Serin bestehenden Gruppe ausgewählt, bevorzugt Lysin ist.

7. Peptid-Lipid-Nanogerüst nach Anspruch 2, wobei das mindestens eine Homing-Molekül mindestens ein aktiver Zelloberflächen-Rezeptorligand ist.

8. Peptid-Lipid-Nanogerüst nach Anspruch 7, ferner umfassend
e) mindestens einen aktiven Wirkstoff,
wobei der aktive Wirkstoff nach der Aufnahme durch die Zellen über den vom SR-B1-Rezeptor vermittelten Pfad in das Zytosol der Zielzelle freigesetzt wird.

9. Peptid-Lipid-Nanogerüst nach Anspruch 8, wobei das Peptid ein Analogon einer amphipathischen α-Helix ist.

10. Peptid-Lipid-Nanogerüst nach Anspruch 8, wobei das Peptid aus der aus 2F, 4F, der Umkehrsequenz von 2F und der Umkehrsequenz von 4F bestehenden Gruppe ausgewählt ist.

11. Peptid-Lipid-Nanogerüst nach Anspruch 8, wobei die/das mindestens eine amphipathische α-Helix/Peptid eine Länge zwischen 8 und 28 Aminosäuren, zwischen 10 und 24 Aminosäuren, zwischen 11 und 22 Aminosäuren, zwischen 14 und 21 Aminosäuren, zwischen 16 und 20 Aminosäuren oder 18 Aminosäuren hat.

12. Peptid-Lipid-Nanogerüst nach Anspruch 8, wobei das Phospholipid aus der aus Phosphatidylcholin (bevorzugt DMPC (1,2-Dimyristoyl-sn-glyzero-3-phosphocholin), POPC (1-Palmitoyl-1-oleyl-phosphatidylcholin) und EYPC (Eigelb-phosphatidylcholin)), Lysophosphatidylcholin, Phosphatidylethanolamin, Phosphatidylserin, Phosphatidylinosit und Kombinationen aus den Vorstehenden bestehenden Gruppe ausgewählt ist.

13. Peptid-Lipid-Nanogerüst nach Anspruch 8, wobei das Nanogerüstes einen Durchmesser zwischen 5-50 nm, zwischen 5-40 nm, zwischen 5-30 nm, zwischen 5-25 nm, zwischen 5-20 nm oder zwischen 10-15 nm hat.

14. Peptid-Lipid-Nanogerüst nach Anspruch 8, wobei der mindestens eine Wirkstoff an mindestens einen Lipidanker gebunden ist, vorzugsweise aus der aus einer Cholesterinoleatgruppe, Cholesterinlaureatgruppe, Phytolgruppe und bevorzugt einer Oleatgruppe und einer ungesättigten Cholesterinestergruppe bestehenden Gruppe ausgewählt und bevorzugt über eine Esterbindung gebunden ist.

15. Peptid-Lipid-Nanogerüst nach Anspruch 8, wobei das Homing-Molekül aus der aus einem Peptid, einem Aptamer, einem Antikörper und einem Antikörperfragment bestehenden Gruppe ausgewählt ist.

16. Peptid-Lipid-Nanogerüst nach Anspruch 8, wobei der aktive Wirkstoff eine lipophile Verbindung ist.

17. Peptid-Lipid-Nanogerüst nach Anspruch 8, wobei der aktive Wirkstoff ein Molekül ist, das einen lipophilen und einen hydrophilen Bestandteil umfasst.

18. Peptid-Lipid-Nanogerüst nach Anspruch 8, wobei der aktive Wirkstoff ein diagnostischer Wirkstoff, ein bildgebender Wirkstoff oder ein therapeutischer Wirkstoff ist.

19. Peptid-Lipid-Nanogerüst nach Anspruch 1, wobei die Aminosäuresequenz konsekutive Aminosäuren eines Apoproteins in Vorwärts-oder Rückwärts-Anordnung, bevorzugt ausgewählt aus der aus apoB-100, apoB-48, apoC, apoE und apoA bestehenden Gruppe, umfasst.

20. Peptid-Lipid-Nanogerüst nach Anspruch 1, ferner eines oder mehrere Triglyceride, Sterine, Sterinester oder Kombinationen aus diesen umfassend.

21. Peptid-Lipid-Nanogerüst nach Anspruch 1, wobei der ungesättigte Lipidester ein ungesättigter Sterinester ist.

22. Peptid-Lipid-Nanogerüst nach Anspruch 21, wobei der ungesättigte Sterinester ein ungesättigter Cholesterinester ist.

23. Peptid-Lipid-Nanogerüst nach Anspruch 22, wobei der ungesättigte Cholesterinester Cholesterinoleat ist.

## Revendications

1. Nano échafaudage peptidique-lipidique apparaissant de façon non naturelle susceptible d'être capté par des cellules par la voie médiée par le récepteur SR-BI comprenant :
a) au moins un phospolipide,
b) au moins un ester lipidique insaturé, et
c) au moins un peptide renfermant entre 6 et 30 acides aminés, ce peptide renfermant une séquence d'acides aminés susceptible de former au moins une hélice alpha amphipatique,
les composants a, b, et c, s'associant pour former le nano échafaudage peptidique-phospholipidique.

2. Nano échafaudage peptidique-lipidique conforme à la revendication 1,
comprenant :
d) au moins une molécule de domiciliation,
les composants a, b, c, et d s'associant pour former le nano échafaudage peptidique-phospholipidique.

3. Nano échafaudage peptidique-lipidique conforme à la revendication 2,
dans lequel la molécule de domiciliation est liée par une liaison covalente au phospholipide.

4. Nano échafaudage peptidique-lipidique conforme à la revendication 2,
dans lequel la molécule de domiciliation est liée au peptide par une liaison covalente.

5. Nano échafaudage peptidique-lipidique conforme à la revendication 4,
dans lequel la molécule de domiciliation est liée au peptide au niveau d'un acide aminé ou à proximité de la transition entre le côté hydrophile et le côté hydrophobe de l'hélice alpha amphipathique.

6. Nano échafaudage peptidique-lipidique conforme à la revendication 5,
dans lequel l'acide aminé du peptide lié à la molécule de domiciliation est choisi dans le groupe formé par la lysine, la cystéine, la thréonine et la sérine et est de préférence la lysine.

7. Nano échafaudage peptidique-lipidique conforme à la revendication 2,
dans lequel la molécule de domiciliation est au moins un ligan d du récepteur de la surface cellulaire actif.

8. Nano échafaudage peptidique-lipidique conforme à la revendication 7,
renfermant en outre :
e) au moins un agent actif,
cet agent actif étant libéré dans le egtosol de la cellule cible après avoir été capté par des cellules par la voie médiée par le récepteur SR-B1.

9. Nano échafaudage peptidique-lipidique conforme à la revendication 8,
dans lequel le peptide est un analogue d'une hélice alpha amphypathique.

10. Nano échafaudage peptidique-lipidique conforme à la revendication 8,
dans lequel peptide est choisi dans le groupe formé par les peptides suivantes : 2F, 4F, la séquence inverse de 2F et la séquence inverse de 4F.

11. Nano échafaudage peptidique-lipidique conforme à la revendication 8,
dans lequel l'hélice alpha amphypathique ou le peptide renferme entre 8 et 28 acides aminés, entre 10 et 24 acides aminés, entre 11 et 22 acides aminés, entre 14 et 21 acides aminés, entre 16 et 20 acides aminés ou 18 acides aminés.

12. Nano échafaudage peptidique-lipidique conforme à la revendication 8,
dans lequel le phospholipide est choisi dans le groupe formé par la phosphatidylcholine (de préférence la DMC (1,2-dimyristoyl-sn-glyco-3-phosphocholine), la POPC (1-palmitoyl-1-olcoyl-phosphatidycholine), et la EYPC (egg yolk phosphatihylcholine)), la lysophosphatidylcholine, la phosphatidylethanolamine, la phosphatidylserine, le phosphatidylinositol et leurs combinaisons.

13. Nano échafaudage peptidique-lipidique conforme à la revendication 8,
ayant un diamètre compris entre 5 et 50nm, entre 5 et 40 nm, entre 5 et 30nm, entre 5 et 25 nm, entre 5 et 20 nm ou entre 10 et 15nm.

14. Nano échafaudage peptidique-lipidique conforme à la revendication 8,
dans lequel l'agent actif est lié à au moins un ancrage de lipide de préférence choisi dans le groupe formé par un fragment cholesterol oleate, un fragment cholesteryl laurate, un fragment phytol et de préférence un fragment oleate et un fragment cholesterol-ester insaturé et de préférence lié par une liaison ester.

15. Nano échafaudage peptidique-lipidique conforme à la revendication 8,
dans lequel la molécule de domiciliation est choisie dans le groupe formé par une peptide, un aptamer, un anti-corps et un fragment d'anti-corps.

16. Nano échafaudage peptidique-lipidique conforme à la revendication 8,
dans lequel l'agent actif est un composé lipophile.

17. Nano échafaudage peptidique-lipidique conforme à la revendication 8,
dans lequel l'agent actif est une molécule renfermant un composé lipophile et un composé hydrophile.

18. Nano échafaudage peptidique-lipidique conforme à la revendication 8,
dans lequel l'agent actif est un agent de diagnostic un agent d'imagerie ou un agent thérapeutique.

19. Nano échafaudage peptidique-lipidique conforme à la revendication 1,
dans lequel la séquence d'acides aminés renferme des acides aminés consécutifs d'une apoprotéine en sens avant ou inverse, de préférence choisie dans le groupe formé par les apoprotéines apoB-100, apoB-48, apoC, apoE et apoA.

20. Nano échafaudage peptidique-lipidique conforme à la revendication 1,
comprenant un triacylglycerol et/ou un stérol et/ou un stérol ester et/ou leurs combinaisons.

21. Nano échafaudage peptidique-lipidique conforme à la revendication 1,
dans lequel l'ester lipidique insaturé est un ester de stérol insaturé.

22. Nano échafaudage peptidique-lipidique conforme à la revendication 21,
dans lequel l'ester de stérol insaturé est un ester de cholestérol insaturé.

23. Nano échafaudage peptidique-lipidique conforme à la revendication 22,
dans lequelle d'ester de cholesthérol insaturé est le cholestéryloleate.
